# EUROPÄISCHE PATENTSCHRIFT

(11) **EP 3 515 277 B1**
(45) Veröffentlichungstag und Bekanntmachung des Hinweises auf die Patenterteilung: **20.07.2022**
(21) Anmeldenummer: 17768749.8
(22) Anmeldetag: 13.09.2017
(51) Int. Cl.: A61B 1/00, B25J 1/02, B25J 13/08, A61B 90/50

(54) **MEDIZINISCHER HALTEARM ZUR EINBINDUNG IN EIN OP-NAVIGATIONSSYSTEM**
MEDICAL HOLDING ARM FOR INCORPORATION INTO A SURGICAL NAVIGATION SYSTEM
BRAS DE RETENUE MÉDICAL DESTINÉ À ÊTRE INTÉGRÉ DANS UN SYSTÈME DE NAVIGATION OPÉRATOIRE

(30) Priorität: 26.09.2016 DE 102016118123
(43) Veröffentlichungstag der Anmeldung: 31.07.2019
(73) Patentinhaber: Brainlab Robotics GmbH, 81829 München (DE)
(72) Erfinder: KRINNINGER, Maximilian, 82234 Weßling-Oberpfaffenhofen (DE); NOWATSCHIN, Stephan, 81677 München (DE)
(74) Vertreter: Eisenführ Speiser
(86) Internationale Anmeldenummer: PCT/EP2017/072965
(87) Internationale Veröffentlichungsnummer: WO 2018/054729

(56) Entgegenhaltungen:
- EP-A1- 1 216 651
- EP-A1- 1 854 425
- EP-A1- 2 821 024
- DE-A1-102014 016 823
- US-A1- 2014 039 517

## Beschreibung

Die Erfindung betrifft eine Haltevorrichtung, insbesondere Haltearm und/oder Stativ, für medizinische Zwecke, insbesondere zum Halten von chirurgischen mechatronischen Assistenzsystemen und/oder chirurgischen Instrumenten. Weiterhin betrifft die Erfindung ein Verfahren.

Haltearme, die unter Haltevorrichtungen der eingangs genannten Art fallen, sind bereits seit längerem bekannt und werden in der Chirurgie insbesondere dazu eingesetzt, einen Operateur von statischer Haltearbeit zu entlasten. Ein derartiger Haltearm wird eingesetzt, um ein mechatronisches Assistenzsystem und/oder ein chirurgisches Instrument zu halten, wie etwa ein Manipulator, ein Endoskop, eine chirurgische Klemme und dergleichen. Insbesondere zum Halten von Endoskopen haben sich die eingangs genannten Haltearme bewährt. Bei der endoskopischen Chirurgie bedient ein Operateur in der Regel mit beiden Händen ein Instrument, während ein Assistent das Endoskop hält, um das Operationsfeld über einen Bildschirm sichtbar zu machen. Das Halten des Endoskops über einen längeren Zeitraum ist sehr ermüdend. Aus diesem Grund werden vermehrt vorgenannte Haltearme eingesetzt.

Ein solcher Haltearm ist beispielsweise aus DE 195 26 915 B4 bekannt. Die dort offenbarte Haltevorrichtung für medizinische Zwecke weist ein Anschlussteil und einen Halter für chirurgische Werkzeuge sowie einen zwischen dem Halter und dem Anschlussteil angeordneten Arm auf. Der Arm ist mit dem Halter und dem Anschlussteil oder mit einem benachbarten Arm über ein Gelenk verbunden und mit einer pneumatisch betätigbaren Vorrichtung zur wahlweisen Festlegung und Lösung der Gelenke überkoppelt, wobei diese Vorrichtung die Gelenke unter Einwirkung einer Bremskraft auf das Gelenk ausübenden mechanischen Feder festlegt und wobei die Vorrichtung gegen die Kraft dieser Feder pneumatisch in einen das Gelenk freigebenden Zustand überführbar ist. An dem proximalen Ende des Arms ist ein Betätigungsorgan angeordnet, mittels dessen Hilfe ein Ventil öffenbar ist, sodass die einzelnen Gelenke des Arms verstellt werden können. Bei Loslassen des Betätigungsorgans wird das Ventil wieder geschlossen, sodass die Gelenke festgelegt sind. Nachteilig ist, dass mit dem Haltearm alle Gelenke gleichzeitig geöffnet werden, wodurch eine Positionierung schwierig sein kann.

Ein ähnlicher Haltearm ist in EP 1 958 587 B1 offenbart. Der dort offenbarte Haltearm weist ebenfalls mehrere Gelenke auf, und zur Betätigung der Gelenke ist ein berührungssensitiver Sensor vorgesehen. Der Sensor ist am Haltearm benachbart zum medizinischen Instrument angeordnet, sodass bei Ergreifen des medizinischen Instruments der Operator in Kontakt kommt mit dem berührungssensitiven Sensor, wodurch alle Gelenke des Haltearms freigegeben werden. Auch hier tritt das oben genannte Problem der mangelhaften Positionierung auf.

Weiterhin besteht bei beiden oben genannten Haltearmen das Problem, dass der Bediener darüber im Unklaren ist, ob tatsächlich alle Gelenke geöffnet sind, wie weit diese geöffnet sind, und welche Bewegungen zulässig sind.

Ferner kommen in der Chirurgie vermehrt robotische Systeme zum Einsatz, die beispielsweise durch einen derartigen Haltearm gehalten werden. In modernen OP-Umgebungen werden chirurgische OP-Navigationssysteme verwendet, die elektromagnetische Strahlung verwenden, insbesondere Infrarotstrahlung oder ein elektromagnetisches Feld. Es kann dabei vorteilhaft sein, wenn das robotische System zum Navigationssystem registriert wird. Hierzu muss das robotische System für das Navigationssystem "sichtbar" sein. Üblicherweise werden hierzu Infrarotreflektoren eingesetzt, die eine bestimmte Geometrie haben und als Tracker bekannt sind. Die Tracker werden an allen zu navigierenden Objekten wie Instrumente, Geräte und Zielgebiet (Patienten) befestigt. Das OP-Navigationssystem kann dann anhand der Reflexion von IR-Strahlung mittels einer Kamera die relative Lage der Instrumente zum Patienten oder einen CT-Datensatz erkennen. Die Tracker haben in der Regel drei Reflexionspunkte, und mittels Triangulation kann über die zwei Kameras des Navigationssystems die Lage und Orientierung des Trackers im Raum, innerhalb des Koordinatensystems der Kamera, erfolgen.

US 2014/0039517 A1 offenbart ein Navigationssystem, das mit einem OP-Roboter verwendet werden kann. Der Roboter kann in einem manuellen oder semi-autonomen Modus genutzt werden. Das Navigationssystem verfolgt den Roboter über Tracker an dem Roboter.

Aus DE 10 2014 016 823 A1 der hiesigen Anmelderin ist ein Haltearm bekannt. Der Haltearm hat ein proximales Ende zum Befestigen des Haltearms an einer Basis und ein distales Ende zum Aufnahmen des chirurgischen mechatronischen Assistenzsystems. Ferner hat er zwei oder mehr Armsegmente und zwei oder mehr Gelenke, mittels denen die Armsegmente gelenkig miteinander verbunden sind, wobei jedes Gelenk mittels einer Bedieneinrichtung freigebbar und arretierbar ist. Der Haltearm hat ferner eine erste Schnittstelle an dem proximalen Ende zum Verbinden des Haltearms mit einer Energiequelle sowie mit einer externen Steuereinheit zum Übertragen von Signalen an den und von dem Haltearm, eine zweite Schnittstelle an dem distalen Ende zum Koppeln des Haltearms mit dem Assistenzsystem zum Steuern des Assistenzsystems, und eine Übertragungseinrichtung, welche innerhalb des Haltearms angeordnet ist und die erste Schnittstelle mit der zweiten Schnittstelle zum Übertragen von Energie und Signalen zwischen den Schnittstellen verbindet.

Aufgabe der vorliegenden Erfindung ist es, die Sicherheit im OP in Bezug auf Haltearme weiter zu verbessern und deren Einsatz zu vereinfachen.

Die Erfindung löst die Aufgabe durch eine Haltevorrichtung mit den Merkmalen des Anspruchs 1, insbesondere eine Haltevorrichtung für medizinische Zwecke, zum Halten eines Anbaugeräts, mit einem proximalen Ende zum Befestigen der Haltevorrichtung an einer Basis und einem distalen Ende zum Aufnehmen eines Anbaugeräts, wenigstens einem ersten und einem zweiten Armsegment, wobei das erste Armsegment mit einem ersten Gelenk und das zweite Armsegment mit einem zweiten Gelenk verbunden ist, wobei jedes Gelenk freigebbar und arretierbar ist, einer Bedieneinrichtung zum Freigeben und/oder Arretieren des entsprechenden Gelenks zum Verbringen der Haltevorrichtung in eine Gewünschte Pose, und einer Steuereinheit, umfassend Hardware und Programmcodemittel zum Steuern der Haltevorrichtung, wobei die Haltevorrichtung ferner wenigstens einen Empfänger für elektromagnetische Strahlung aufweist, der mit der Steuereinheit verbunden ist, und dazu angepasst ist, basierend auf empfangenen elektromagnetischen Signalen eines OP-Navigationssystems Signale an die Steuereinheit zu senden. Vorzugsweise ist die Haltevorrichtung in einem Navigationsmodus und einem Bedienmodus betreibbar ist, wobei die Haltevorrichtung in dem Bedienmodus rein manuell über eine für die Haltevorrichtung vorgesehene Bedieneinheit bedienbar ist, und in dem Navigationsmodus dazu vorbereitet ist, Befehle von dem Navigationssystem zu empfangen, wobei die Steuereinheit dazu eingerichtet ist basierend auf dem empfangenen Signal zu bestimmen, dass sich die Haltevorrichtung in einer navigierten OP-Umgebung befindet und in den Navigationsmodus zu schalten.

Der Erfindung liegt die Erkenntnis zugrunde, dass sich durch einen derartigen Empfänger die Haltevorrichtung besonders einfach und zweckmäßig in eine bestehende OP-Navigationsumgebung einbinden lässt. OP-Navigationssysteme nutzen in der Regel Infrarot- oder elektromagnetische Strahlung, um einzelne im OP-Bereich befindliche Objekte zu identifizieren. Die Haltevorrichtung empfängt über den Empfänger die elektromagnetische Strahlung und sendet ein Signal an die Steuereinheit. Die Steuereinheit bestimmt, vorzugsweise basierend auf dem empfangenen Signal, dass sich die Haltevorrichtung in einer navigierten OP-Umgebung befindet. Das heißt, bei Empfang der elektromagnetischen Strahlung, erkennt die Haltevorrichtung selbsttätig, dass sie sich in einem Sichtfeld des OP-Navigationssystems bzw. der Kameras eines solchen Systems befindet, ohne dass es hierzu eines zusätzlichen externen Signals, wie beispielsweise eine Bedienereingabe oder ein Statussignal des OP-Navigationssystems das drahtgebunden oder drahtlos übertragen wird, bedarf. Die Haltevorrichtung ist also in der Lage autonom zu erkennen, wann sie sich im Sichtfeld des OP-Navigationssystems befindet.

Die Haltevorrichtung weist vorzugsweise wenigstens einen Navigationsmodus und einen Bedienmodus auf, wobei die Haltevorrichtung in dem Bedienmodus rein manuell, d.h. durch manuelle Berührung, über eine für die Haltevorrichtung vorgesehene Bedieneinheit, wie etwa ein spezieller Laptop, Fernbedienung oder dergleichen, oder über einen angeschlossenen Computer bedienbar ist. In dem Navigationsmodus ist die Haltevorrichtung vorzugsweise mit dem Navigationssystem gekoppelt und dazu vorbereitet, Befehle von dem Navigationssystem zu empfangen. Wird durch die Steuereinheit bestimmt, dass die Haltevorrichtung in einer navigierten OP-Umgebung ist, schaltet die Steuereinheit vorzugsweise in den Navigationsmodus. Im Navigationsmodus kann vorgesehen sein, dass verschiedene Funktionen der Haltevorrichtung geändert sind, wie beispielsweise Maximalwinkel an den Gelenken und/oder das Freigeben oder Arretieren der entsprechenden Gelenke. Beispielsweise kann vorgesehen sein, dass die Bedieneinrichtung in diesem Fall gesperrt ist und eine manuelle Bedienung der Haltevorrichtung nicht gestattet ist.

Die Haltevorrichtung muss aber nicht zwangsläufig von dem Navigationssystem gesteuert werden. Die Erfindung basiert auf der Idee, dass die Haltvorrichtung autonom erkennt, dass sie in einer navigierten Umgebung ist. Eine Änderung an einem bestehenden OP-Navigationssystem ist dafür nicht erforderlich. Auch muss das OP-Navigationssystem nicht besonders für den Betrieb in Verbindung mit der Haltevorrichtung gemäß der vorliegenden Erfindung ausgebildet oder vorbereitet sein. Allein basierend auf dem Empfang der elektromagnetischen Strahlung des OP-Navigationssystems erkennt die Haltevorrichtung, dass sie in deren Blickfeld ist. In diesem Fall sendet der Empfänger ein entsprechendes Signal an die Steuereinheit. Der Empfänger ist Drahtlosempfänger.

Die Haltevorrichtung weist ferner im Inneren ein BUS-System sowie eine erste mechatronische Schnittstelle an dem proximalen Ende und eine zweite mechatronische Schnittstelle an dem distalen Ende auf. An der Schnittstelle am proximalen Ende ist die Haltevorrichtung vorzugsweise verbindbar mit dem OP-Navigationssystem zur Datenübertragung sowie weiteren Systemen, wie beispielsweise einem CAM-System, oder einer Dokumentationseinrichtung eines OPs. An dem distalen Ende nimmt die Haltevorrichtung vorzugsweise ein Anbaugerät auf, welches insbesondere ein robotisches Anbaugerät sein kann. Ein robotisches Anbaugerät umfasst insbesondere einen Manipulator mit einem oder mehreren Aktuatoren, insbesondere Stellmotoren, und ist dazu vorgesehen, ein chirurgisches Instrument, wie beispielsweise ein Endoskop oder dergleichen, zu bewegen und zu führen.

Über den Empfänger ist die Haltevorrichtung vorzugsweise dazu eingerichtet, Stellsignale zu empfangen, die über die Steuereinheit und das interne BUS-System vorzugsweise über die Schnittstelle am distalen Ende an ein aufgenommenes Anbaugerät, insbesondere robotisches Anbaugerät, geleitet werden. Auf diese Weise ist es möglich, dass die Haltevorrichtung über das Navigationssystem drahtlos, nämlich über die elektromagnetische Strahlung, Stellsignale für das robotische Anbaugerät erhält. Hierdurch ist das Einbinden der Haltevorrichtung in eine navigierte OP-Umgebung besonders einfach.

Vorzugsweise weist der Empfänger einen Infrarotsensor auf. Ein solcher Infrarotsensor ist vorzugsweise als Infrarotfotodiode ausgebildet. Vorzugsweise weist der Empfänger eine Mehrzahl an Infrarotsensoren auf. Bevorzugt ist wenigstens an jedem Armsegment und/oder an jedem Gelenk ein Infrarotsensor vorgesehen. Vorzugsweise sind 2, 3, oder 4 Infrarotsensoren um den Umfang eines jeden Armsegments herum angeordnet. Hierdurch ist es möglich, dass unabhängig von der Pose der Haltevorrichtung das Infrarotsignal des OP-Navigationssystems empfangen wird. In einer Variante ist nur an einem Armsegment, beispielsweise an dem mittleren oder an dem distalen, um den Umfang herum eine Mehrzahl an Infrarotsensoren vorgesehen. Dienen die Infrarotsensoren nur dazu, Signale zu empfangen und an eine zentrale Steuereinheit der Haltevorrichtung weiterzugeben, reicht es aus, dass für eine zentrale Kommunikation nur an einem Armsegment derartige Infrarotsensoren vorgesehen sind. Alternativ ist auch denkbar, dass der Empfänger ein oder mehrere CCD-Chips aufweist.

In einer bevorzugten Weiterbildung weist der Empfänger einen Hall-Sensor auf. Anstelle eines Hall-Sensors kann auch ein anderer, geeigneter Sensor vorgesehen sein, der dazu angepasst ist, ein elektromagnetisches Feld zu erfassen. Mittels Hall-Sensoren ist es möglich, die Stärke eines elektromagnetischen Felds zu erfassen, und so Signale aus einem Navigationssystem, das mit elektromagnetischen (EM) Feldern arbeitet und einen EM-Feldgenerator aufweist, zu kommunizieren. Der Hall-Sensor sendet bei Empfang des elektromagnetischen Felds ein entsprechendes Signal, sodass das entsprechende Armsegment, an dem der Hall-Sensor vorgesehen ist, erkennt, sobald sich das entsprechende Segment im Arbeitsvolumen des EM-Feldgenerators befindet.

Gemäß einer weiteren bevorzugten Ausführungsform weist der Empfänger ein 3D-Magnetometer auf. Vorzugsweise sind in zwei oder mehr Armsegmenten, vorzugsweise allen Armsegmenten der Haltevorrichtung, ein 3D-Magnetometer angeordnet. Durch derartige 3D-Magnetometer ist es möglich, die Lage des entsprechenden Armsegments, in dem das Magnetometer angeordnet ist, in dem elektromagnetischen Feld des EM-Feldgenerators zu bestimmen. Basierend auf Daten, die die Steuereinheit von dem oder den 3D-Magnetometern erhält, ist die Pose der Haltevorrichtung bestimmbar. Diese Pose, die der Haltearm selbst bestimmt, kann dann mittels der Steuereinheit und über das interne BUS-System an die Schnittstelle am proximalen Ende gegeben werden, und von dort aus kabelgebunden, oder drahtlos an das OP-Navigationssystem weitergegeben werden. Basierend hierauf kann ein Vergleich zwischen einer Pose, die das OP-Navigationssystem aufgrund einer eigenen Erfassungseinrichtung bestimmt hat und der von der Haltevorrichtung selbst bestimmten Pose durchgeführt werden. Hierdurch können Berechnungsfehler ausgeglichen werden, indem die beiden Posen abgeglichen werden. Hierdurch ist die Sicherheit weiter verbessert.

Gemäß einer weiteren bevorzugten Ausgestaltung der Erfindung weist die Haltevorrichtung wenigstens einen Sender zum Senden elektromagnetischer Strahlung zum Senden von Signalen an ein OP-Navigationssystem auf. Ein derartiger Sender zum Senden elektromagnetischer Strahlung weist vorzugsweise einen aktiven Sender auf. Ebenso ist bevorzugt, dass er einen passiven Sender aufweist. Als passiver Sender wird vorliegend insbesondere ein Reflektor verstanden, der elektromagnetische Strahlung, die von OP-Navigationssystemen emittiert wurde, reflektiert. Als aktiver Sender wird ein Sender verstanden, der selbstemittierend ist, also etwa eine IR-LED.

Mittels eines derartigen Senders ist es nicht nur möglich, dass die Haltevorrichtung Signale von OP-Navigationssystemen empfängt, sondern ebenso Signale an das OP-Navigationssystem sendet. Derartige Signale umfassen insbesondere die Lage von einem oder mehreren Armsegmenten der Haltevorrichtung, als auch einen Status der Haltevorrichtung. Beispielsweise ist es bevorzugt, dass ein oder mehrere aktive Sender dazu ausgebildet sind, ein Signal zu emittieren, wenn die Haltevorrichtung in einen bestimmten Status wechselt. Beispielsweise sind der eine oder die mehreren aktiven Sender dazu ausgebildet, ein Signal auszugeben, wenn eine oder mehrere Bremsen an den Gelenken geöffnet werden und/oder ein oder mehrere Gelenke bewegt werden. Ebenso ist es möglich, dass der eine oder die mehreren aktiven Sender mittels elektromagnetischer Signale die selbstbestimmte Pose der Haltevorrichtung senden. Hierdurch ist eine drahtlose Datenübertragung zwischen Haltevorrichtung und OP-Navigationssystem erreicht, die keine zusätzlichen Vorrichtungen, wie beispielsweise WLAN oder dergleichen nutzt. Vielmehr wird das dem Navigationssystem immanente Übermittlungsverfahren genutzt, nämlich insbesondere Infrarot-Strahlung oder elektromagnetische Strahlung. Vorzugsweise weist der Sender wenigstens eine Infrarotlichtquelle auf. Vorzugsweise ist die Infrarotlichtquelle als Infrarot-LED ausgebildet. Vorzugsweise sind mehrere, d.h. zwei oder mehr, dieser Infrarotlichtquellen vorgesehen.

Besonders bevorzugt weist die Haltevorrichtung eine erste Anzeigeeinheit, die an dem ersten Gelenk angeordnet ist und eine zweite Anzeigeeinheit, die an dem zweiten Gelenk angeordnet ist auf, wobei die erste und/oder zweite Anzeigeeinheit jeweils wenigstens eine IR-Lichtquelle aufweisen und dazu eingerichtet sind, wenigstens einen Status der Haltevorrichtung und/oder einen Status eines Anbaugeräts anzuzeigen. Die Anzeigeeinheiten weisen neben den IR-Lichtquellen vorzugsweise auch Lichtquellen auf, die Licht im sichtbaren Bereich emittieren, wie insbesondere LEDs. Ferner ist bevorzugt, dass die Anzeigeeinheiten IR-Fotodioden aufweisen, um infrarote Strahlung zu empfangen, wobei die IR-Fotodioden Teil des Empfängers sind.

Die Anzeigeeinheiten sind vorzugsweise ringförmig und koaxial zu Rotationsachsen der Gelenke angeordnet. Die genaue Ausgestaltung der Anzeigeeinheiten ist in PCT/EP2016/069167 beschrieben. Die Anzeigeeinheiten zeigen auch für einen Bediener visuell wahrnehmbar den Status der Haltevorrichtung an. Hierdurch ist durch einen Bediener unmittelbar erkennbar, welche Signale, bzw. was für Informationen die Haltevorrichtung an das OP-Navigationssystem sendet.

Gemäß einer weiteren bevorzugten Ausführungsform sind der eine oder die mehreren aktiven Sender dazu ausgebildet, ein Signal auszugeben, wenn eine oder mehrere Bremsen an den Gelenken der Haltevorrichtung geöffnet werden und/oder ein oder mehrere Gelenke der Haltevorrichtung bewegt werden. die Haltevorrichtung weist vorzugsweise Bremsen an den Gelenken auf, die mittels einer Bedieneinrichtung geöffnet werden können. Werden diese Bremsen geöffnet, kann die Haltevorrichtung bewegt werden, das heißt in ihrer Pose verändert werden. Der eine oder die mehreren aktiven Sender geben in diesem Fall vorzugsweise ein Signal ab. Es kann auch vorgesehen sein, dass ein Signal nur dann abgegeben wird, wenn eines oder mehrere Gelenke bewegt werden. Allein durch das Öffnen einer Bremse wird die Pose der Haltevorrichtung noch nicht verändert. Entscheidend ist vielmehr, dass eines der Gelenke bewegt wird. Daher ist bevorzugt, dass in diesem Fall ein Signal ausgegeben wird. Auf diese Weise ist es möglich, dass die Haltevorrichtung dem OP-Navigationssystem eine bevorstehende Bewegung, oder eine tatsächlich stattfindende Bewegung mitteilt. Vorzugsweise repräsentiert das Signal das eine oder die mehreren freigegebenen Gelenke. Vorzugsweise repräsentiert das Signal die eine oder die mehreren Bewegungen der einen oder mehreren Gelenke, vorzugsweise Geschwindigkeit und/oder Beschleunigung. Vorzugsweise repräsentiert das Signal nach Abschluss der Bewegung und/oder Zusperren der einen oder der mehreren Bremsen, eine dann eingenommene Pose der Haltevorrichtung.

Gemäß einer weiteren bevorzugten Ausführungsform weist die Haltevorrichtung eine Navigationskamera zum Erfassen eines Operationsfelds auf. Vorzugsweise ist die Haltevorrichtung dazu eingerichtet, von der Navigationskamera erfasste Signale an einer Schnittstelle für das OP-Navigationssystem bereitzustellen. Die Schnittstelle kann eine physische Schnittstelle an dem proximalen Ende der Haltevorrichtung sei, sodass die Signale kabelgebunden an das OP-Navigationssystem bereitgestellt werden. Vorzugsweise werden die von der Navigationskamera erfassten Signale drahtlos über einen Sender zum Senden elektromagnetischer Strahlung zum Senden von Signalen an ein OP-Navigationssystem bereitgestellt. Auf diese Weise ist es möglich, dass die Haltevorrichtung selbst mittels der eigenen Navigationskamera das Operationsfeld beobachtet. Es kann vorkommen, dass die Haltevorrichtung das OP-Feld oder einen Teil davon gegenüber dem OP-Navigationssystem und der Kamera des OP-Navigationssystems abschattet. Weist die Haltevorrichtung selbst eine Navigationskamera auf, kann auch vom abgeschatteten Bereich eine Aufnahme gemacht werden und diese an das OP-Navigationssystem bereitgestellt werden. Das OP-Navigationssystem kann dann die Signale von der dem OP-Navigationssystem zugeordneten Navigationskamera sowie der Navigationskamera an dem Haltearm fusionieren, um so ein Gesamtbild des OP-Felds zu erhalten, ohne Abschattung durch die Haltevorrichtung.

Die von der Navigationskamera erfassten Daten können prozessiert oder unprozessiert bereitgestellt werden. Bei einer unprozessierten Bereitstellung der Daten werden diese lediglich von der Navigationskamera erfasst und dann an einer entsprechenden Schnittstelle der Haltevorrichtung bereitgestellt. Die prozessierten Daten können darüber hinaus zu den Ortsdaten, Daten über die Pose der Haltevorrichtung sowie einen Ort der Navigationskamera und Blickrichtung der Navigationskamera der Haltevorrichtung umfassen. Auch ist es denkbar, dass prozessierte Daten zusätzlich Informationen über in dem OPFeld befindliche Objekten enthalten, die die Navigationskamera an der Haltevorrichtung erfasst hat.

Bevorzugt ist weiterhin, dass die Haltevorrichtung ein Bussystem sowie eine erste mechatronische Schnittstelle an dem proximalen Ende und eine zweite mechatronische Schnittstelle am distalen Ende aufweist, wobei die mechatronische Schnittstelle an dem distalen Ende zum Koppeln mit einem robotischen Anbaugerät vorgesehen ist. Über die erste mechatronische Schnittstelle an dem proximalen Ende können auch die Daten der Navigationskamera hierüber übertragen werden.

Gemäß einem zweiten Aspekt der Erfindung wird die eingangs genannte Aufgabe gelöst durch ein robotisches Anbaugerät nach Anspruch 7, insbesondere Manipulatorvorrichtung, mit einem Rahmen, einem an dem Rahmen abgestützten Antrieb, einer Instrumentenaufnahme zum Halten eines chirurgischen Instruments, die mittels des Antriebs antreibbar ist, und einer mechatronischen Schnittstelle zum Koppeln mit einer distalen Schnittstelle einer Haltevorrichtung, insbesondere einer Haltevorrichtung nach einer der vorstehend beschriebenen bevorzugten Ausführungsformen einer Haltevorrichtung gemäß dem ersten Aspekt der Erfindung, wobei wenigstens ein Empfänger für elektromagnetische Strahlung vorgesehen ist, der mit einer Roboter-Steuereinheit verbunden ist und dazu angepasst ist, basierend auf empfangenen elektromagnetischen Signalen eines OP-Navigationssystems Signale an die Roboter-Steuereinheit zu senden. Das robotische Anbaugerät ist dazu vorgesehen, mit einer Haltevorrichtung gekoppelt zu werden. Die Aufgabe wird also auch dadurch gelöst, dass das robotische Anbaugerät selbst ermittelt, dass es sich in einer navigierten Umgebung befindet, wie dies grundsätzlich schon mit Bezug auf die Haltevorrichtung gemäß dem ersten Aspekt der Erfindung oben beschrieben wurde. Insofern soll verstanden werden, dass die Haltevorrichtung gemäß dem ersten Aspekt der Erfindung und das robotische Anbaugerät gemäß dem zweiten Aspekt der Erfindung gleiche und ähnlich Unteraspekte aufweisen, wie sie insbesondere in den abhängigen Ansprüchen niedergelegt sind. Insofern wird für bevorzugte Ausführungsformen und Vorteile des Empfängers und der Roboter-Steuereinheit auf dem Empfänger der Haltevorrichtung und die Steuereinheit der Haltevorrichtung gemäß dem ersten Aspekt der Erfindung verwiesen.

Die Roboter-Steuereinheit wirkt vorzugsweise über die mechatronische Schnittstelle mit der Steuereinheit der Haltevorrichtung zusammen und tauscht mit dieser Daten aus, insbesondere Signale, die von dem Empfänger des robotischen Anbaugeräts oder dem Empfänger der Haltevorrichtung empfangen wurden.

Vorzugsweise ist das robotische Anbaugerät in einem Roboter-Navigationsmodus und einem Roboter-Bedienmodus betreibbar, wobei das Anbaugerät in dem Roboter-Betriebsmodus rein manuell über eine für das Anbaugerät vorgesehene Roboter-Bedieneinheit bedienbar ist, und in dem Roboter-Navigationsmodus dazu vorbereitet ist, Befehle von den Navigationssystemen zu empfangen, wobei die Roboter-Steuereinheit dazu eingerichtet ist, basierend auf dem empfangenen Signal zu bestimmen, dass sich das Anbaugerät in einer navigierten OP-Umgebung befindet und in den Roboter-Navigationsmodus zu schalten. Vorzugsweise ist das robotische Anbaugerät dazu eingerichtet, in Antwort auf ein vom Empfänger empfangenes Signal, ein Navigationssignal an der mechatronischen Schnittstelle bereitzustellen. Über die mechatronische Schnittstelle kann das Navigationssignal dann an die Haltevorrichtung übertragen werden, die dieses weiterverarbeitet oder direkt an der proximalen Schnittstelle der Haltevorrichtung bereitstellt. Ein solches Navigationssignal kann dazu verwendet werden, anzuzeigen, dass sich das robotische Anbaugerät in dem Roboter-Navigationsmodus befindet. Ein solches Signal kann aber auch dazu verwendet werden, ein OP-Protokoll automatisiert zu erstellen. Gemäß einer weiteren bevorzugten Ausführungsform weist das robotische Anbaugerät einen ersten Halter und einen zweiten Halter zum Halten einer Instrumentenaufnahme für das chirurgische Instrument auf, eine erste an dem Rahmen gelagerte Lenkeranordnung, welche den Rahmen mit dem ersten Halter gelenkig verbindet, und eine zweite Lenkeranordnung, welche den Rahmen mit dem zweiten Halter gelenkig verbindet, wobei die ersten und zweiten Lenkeranordnung jeweils in zueinander parallelen und beabstandeten ersten und zweiten Bewegungsebenen relativ zum Rahmen bewegbar sind, sodass der erste Halter in der ersten Bewegungsebene und der zweite Halter in der zweiten Bewegungsebene bewegbar ist, wobei die erste Lenkeranordnung an vier Hebelgelenkpunkten der ersten Lenkeranordnung mit dem Rahmen gekoppelt ist, und die zweite Lenkeranordnung an vier Hebelgelenkpunkten der zweiten Lenkeranordnung mit dem Rahmen gekoppelt ist.

Damit ist ein robotisches Anbaugerät geschaffen, das die ersten und zweiten Halter in separaten und stets parallel zueinander angeordneten Bewegungsebenen bewegen kann. Eine Verschwenkung der Ebenen zueinander, wird nicht umgesetzt. Hierdurch kann ein Gelenk in dem Rahmen entfallen, und der Rahmen kann insgesamt steifer ausgebildet werden. Jede Lenkeranordnung ist zudem über vier Hebelgelenkpunkte mit dem Rahmen gekoppelt, wodurch wiederum eine höhere Steifigkeit erreicht wird. Zum reinen Positionieren der ersten und zweiten Halter reichen grundsätzlich zwei Hebelgelenkpunkte je Lenkeranordnung aus. Die jeweils zwei weiteren, die vorzugsweise vorgesehen sind, dienen dann insbesondere zur Stabilisierung.

Vorzugsweise sind die vier ersten Hebelgelenkpunkte und die vier zweiten Hebelgelenkpunkte jeweils V-förmig angeordnet sind. Hierdurch wird weiterhin vermieden, dass die Lenkeranordnungen Singularitäten einnehmen können. Durch die V-förmige Anordnung der vier Hebelgelenkpunkte je Lenkeranordnung wird erreicht, dass jede Position der ersten und zweiten Halter eindeutig ist. Geometrisch oder statisch unbestimmte Positionen werden vermieden. Hierdurch ist insbesondere auch die Sicherheit der chirurgischen Manipulatorvorrichtung wesentlich erhöht, da es während einer Operation nicht zu Singularitäten in der Kinematik kommen kann. Zwar ist grundsätzlich auch eine Anordnung der vier ersten Hebelgelenkpunkte und der vier zweiten Hebelgelenkpunkte jeweils in einem Rechteck im Rahmen der Erfindung bevorzugt, allerdings sollten dann andere Mittel vorgesehen werden, um Singularitäten auszuschließen, wie etwas ein Begrenzen der Bewegungsfreiheit.

Gemäß einem bevorzugten Weiterbildung des zweiten Aspekts der Erfindung weist das robotische Anbaugerät einen Rahmen, einen an dem Rahmen abgestützten Antrieb, eine Instrumentenaufnahme zum Halten eines chirurgischen Instruments, die mittels des Antriebs antreibbar ist, und eine mechatronischen Schnittstelle zum Koppeln mit einer distalen Schnittstelle einer Haltevorrichtung, insbesondere einer Haltevorrichtung nach einer der vorstehend beschriebenen bevorzugten Ausführungsformen einer Haltevorrichtung gemäße dem ersten Aspekt der Erfindung, sowie eine Navigationskamera zum Erfassen eines Operationsfelds auf.

Das robotische Anbaugerät ist vorzugsweise dazu eingerichtet ist, von der Navigationskamera erfasste Signale prozessiert oder unprozessiert an einer Schnittstelle für das OP-Navigationssystem und/oder der mechatronischen Schnittstelle bereitzustellen.

Durch das Vorsehen der Navigationskamera an dem robotischen Anbaugerät kann das Operationsfeld noch besser beobachtet werden. Eine zusätzliche Abschattung durch Elemente des robotischen Anbaugeräts gegenüber der Navigationskamera des Haltearms findet nicht statt. Zudem lässt sich das robotische Anbaugerät robotisch steuern, während der Haltearm vorzugsweise insgesamt passiv ist. Das heißt, es ist denkbar, dass durch Befehle, wie beispielsweise Signale, die mittels elektromagnetischer Strahlung von dem OP-Navigationssystem drahtlos an die Haltevorrichtung und/oder das robotische Anbaugerät übertragen werden, eine Blickrichtung der Navigationskamera an dem robotischen Anbaugerät angepasst, geändert oder auf andere Weise gesteuert wird. Auch kann vorgesehen sein, dass die Navigationskamera an dem robotischen Anbaugerät eine Sprachsteuerung aufweist, dahingehend, dass gesprochene Befehle eines Operateurs in elektrische Signale umgesetzt werden zum Steuern der Navigationskamera. So ist es beispielsweise denkbar, dass ein Operateur das Wort "Snapshot" spricht und die Navigationskamera ein Foto aufnimmt, welches dann über das robotische Anbaugerät, die mechatronische Schnittstelle des robotischen Anbaugeräts an die mechatronische Schnittstelle am distalen Ende der Haltevorrichtung, von dort über das interne Bussystem zur proximalen Schnittstelle der Haltevorrichtung übertragen und dort bereitgestellt wird.

Vorzugsweise weist das robotische Anbaugerät gemäß dem zweiten Aspekt der Erfindung wenigstens einen Sender zum Senden elektromagnetischer Strahlung zum Senden von Signalen an ein OP-Navigationssystem auf. Auch ein solcher Sender zum Senden von elektromagnetischer Strahlung zum Senden von Signalen an ein OP-Navigationssystem wurde bereits oben in Bezug auf die Haltevorrichtung beschrieben. Dennoch ist es auch bevorzugt, dass auch das robotische Anbaugerät einen derartigen Sender aufweist. Das robotische Anbaugerät wird robotisch gesteuert, das heißt ändert seine Pose selbsttätig basierend auf empfangenen elektronischen Signalen.

In einem dritten Aspekt der Erfindung wird die eingangs genannte Aufgabe durch ein System nach Anspruch 11 gelöst, welches eine Haltevorrichtung nach einer der vorstehend beschriebenen bevorzugten Ausführungsformen einer Haltevorrichtung gemäß einem ersten Aspekt der Erfindung sowie ein am distalen Ende aufgenommenes Anbaugerät aufweist, wobei das Anbaugerät wenigstens einen Sender zum Senden elektromagnetischer Strahlung zum Senden von Signalen an ein OP-Navigationssystem aufweist. Das Anbaugerät ist vorzugsweise ein robotischer Manipulator. Vorzugsweise ist der Sender des Anbaugeräts entsprechend dem Sender der Haltevorrichtung aufgebaut und weist vorzugsweise eine oder mehrere IR-Lichtquellen auf.

Gemäß einem Aspekt wird die eingangs genannte Aufgabe bei einem System gelöst, welches eine Haltevorrichtung nach einer der vorstehend beschriebenen bevorzugten Ausführungsformen einer Haltevorrichtung gemäß dem ersten Aspekt der Erfindung aufweist sowie ein am distalen Ende der Haltevorrichtung aufgenommenes Anbaugerät, wobei das Anbaugerät wenigstens einen Empfänger für elektromagnetische Strahlung aufweist.

Vorzugsweise weist das Anbaugerät eine Schnittstelle auf, über die das Anbaugerät mit dem internen BUS-System der Haltevorrichtung koppelbar ist. Sowohl der Sender als auch der Empfänger des Anbaugeräts gemäß den Systemen des zweiten und dritten Aspekts der Erfindung, ist vorzugsweise über das BUS-System mit der Steuereinheit der Haltevorrichtung gekoppelt. Ferner ist bevorzugt, dass das Anbaugerät eine eigene Steuereinheit aufweist. Sowohl Sender als auch Empfänger sind entsprechend mit der Steuereinheit des Anbaugeräts und/oder über das BUS-System mit der Steuereinheit der Haltevorrichtung gekoppelt. Bei dieser Ausführungsform, bei der das Anbaugerät einen Empfänger aufweist, ist es möglich, dass das OP-Navigationssystem mittels der von dem OP-Navigationssystem verwendeten Art der Strahlung, elektromagnetisches Feld oder Infrarotstrahlung, Signale und insbesondere Befehle an das Anbaugerät sendet. Entsprechend kann auch das Anbaugerät Signale, wie insbesondere seinen Status, an das OP-Navigationssystem senden.

Gemäß einem vierten Aspekt der Erfindung wird die eingangs genannte Aufgabe gelöst durch ein Verfahren nach Anspruch 12 zur Kommunikation zwischen einer Haltevorrichtung nach einer der vorstehend beschriebenen bevorzugten Ausführungsformen einer Haltevorrichtung gemäß dem ersten Aspekt der Erfindung und einem OP-Navigationssystem, mit den Schritten: Empfangen von elektromagnetischer Strahlung von einem OP-Navigationssystem mittels des Empfängers; Senden eines entsprechenden Signals von dem Empfänger an die Steuereinheit; Verarbeiten, mittels der Steuereinheit, des von dem Empfänger empfangenen Signals; und Versetzen der Haltevorrichtung in einen Navigationsmodus. Vorzugsweise umfasst der Schritt des Verarbeitens Erkennen, mittels der Steuereinheit, dass sich die Haltevorrichtung in einem Navigationsbereich des OP-Navigationssystems befindet. Die Haltevorrichtung weist vorzugsweise wenigstens einen Navigationsmodus und einen Bedienmodus auf, wobei die Haltevorrichtung in dem Bedienmodus rein manuell, d.h. durch manuelle Berührung, über eine für die Haltevorrichtung vorgesehene Bedieneinheit, wie etwa ein spezieller Laptop, Fernbedienung oder dergleichen, oder über einen angeschlossenen Computer bedienbar ist. In dem Navigationsmodus ist die Haltevorrichtung vorzugsweise mit dem Navigationssystem gekoppelt und dazu vorbereitet, Befehle von dem Navigationssystem zu empfangen. Wird durch die Steuereinheit bestimmt, dass die Haltevorrichtung in einer navigierten OP-Umgebung ist, schaltet die Steuereinheit vorzugsweise in den Navigationsmodus. Alternativ kann auch durch ein Navigationssystem vorgegeben sein, dass sich die Haltevorrichtung in einer navigierten Umgebung befindet. Beispielsweise sendet das Navigationssystem ein entsprechendes Signal über die proximale Schrittstelle der Haltevorrichtung an diese, um die Haltevorrichtung zu veranlassen in den Navigationsmodus zu schalten. Ein selbsttätiges Bestimmen der Haltevorrichtung ist dann nicht erforderlich. Im Navigationsmodus kann vorgesehen sein, dass verschiedene Funktionen der Haltevorrichtung geändert sind, wie beispielsweise Maximalwinkel an den Gelenken und/oder das Freigeben oder Arretieren der entsprechenden Gelenke. Beispielsweise kann vorgesehen sein, dass die Bedieneinrichtung in diesem Fall gesperrt ist und eine manuelle Bedienung der Haltevorrichtung nicht gestattet ist. In dem Navigationsmodus ist vorzugsweise vorgesehen, dass von dem Empfänger an die Steuereinheit gesendete Signale verarbeitet und an der proximalen Schnittstelle zur Übertragung an das Navigationssystem bereitgestellt werden. Der Empfänger kann einen oder mehrere IR-Sensoren, einen oder mehrere Hall-Sensoren, und/oder einen oder mehrere 3D-Magnetometer aufweisen, wobei die Aufzählung nicht abschließend ist.

Weiterhin weist das Verfahren ferner die Schritte auf: Senden, mittels der elektromagnetischen Strahlung, von Befehlen des OP-Navigationssystems an die Haltevorrichtung; und Empfangen der Befehle mittels des Empfängers. Der Empfänger empfängt die Signale und sendet diese an die Steuereinheit, verarbeitet die Signale in dem Prozessor, unter Verwendung der Programmcodemittel und veranlasst einen oder mehrere Aktuatoren der Haltevorrichtung eine oder mehrere Operationen durchzuführen. Zusätzlich oder alternativ sendet die Steuereinheit entsprechende Signale an ein an der distalen Schnittstelle aufgenommenes Anbaugerät, die ein oder mehrere Aktuatoren des Anbaugeräts veranlassen, eine oder mehrere Operationen durchzuführen.

In einer weiteren bevorzugten Ausführungsform des Verfahrens weist die Haltevorrichtung wenigstens einen Sender zum Senden elektromagnetischer Strahlung zum Senden von Signalen an ein OP-Navigationssystem auf, und das Verfahren umfasst die Schritte: Senden von elektromagnetischer Strahlung zum Senden von Signalen an das OP-Navigationssystem mittels des Senders, und Empfangen der Signale an dem OP-Navigationssystem, wobei die Signale einen Status der Haltevorrichtung und/oder eines Anbaugeräts repräsentieren. Hierbei ist es möglich, dass die Haltevorrichtung ihren eigenen Status mittels des Senders dem OP-Navigationssystem mitteilt. Der Sender umfasst vorzugsweise einen oder mehrere Infrarotlichtquellen. Vorzugsweise umfasst das Verfahren Abgeben von Infrarotstrahlung mit einer bestimmten Frequenz, oder mit einem vorbestimmten Pulsschema, zum Senden von Informationen an das OP-Navigationssystem. Das Senden von Informationen mittels eines vorbestimmten Pulsschemas ist analog zu verstehen zu einem Morsen. So ist beispielsweise denkbar, dass die IR-Lichtquelle mit einem vorbestimmten Schema blinkt, um dem OP-Navigationssystem die aktuelle Pose der Haltevorrichtung mitzuteilen. Auch können andere Informationen, wie ein Bereitschaftsstatus, das Öffnen von Gelenken, die Art des aufgenommen Anbaugeräts, und ähnliche mitgeteilt werden.

In einer besonders bevorzugten Ausführungsform des Verfahrens, bei der die Haltevorrichtung wenigstens ein 3D-Magnetometer aufweist und die Haltevorrichtung in einem elektromagnetischen Feld eines OP-Navigationssystems angeordnet ist, umfasst das Verfahren die Schritte: Bestimmen, basierend auf von dem wenigstens einen 3D-Magnetometer gesendeten Signalen, einer relativen Pose der Haltevorrichtung bezogen auf das elektromagnetische Feld; Bestimmen einer absoluten Pose der Haltevorrichtung; Vergleichen der relativen und der absoluten Posen der Haltevorrichtung; und Bestimmen eines Fehlers, mittels einer Fehlerkorrektureinheit, des elektromagnetischen Felds basierend auf dem Vergleich der relativen und der absoluten Pose der Haltevorrichtung. OP-Navigationssysteme, die auf elektromagnetischen Feldern, erzeugt durch EM-Feldgeneratoren, basieren, bringen das Problem mit sich, dass in den Navigationsbereich eingebrachte Objekte, wie insbesondere Instrumente oder dergleichen, Einfluss auf das elektromagnetische Feld haben und daher die Positionsbestimmung dieser Objekte in dem elektromagnetischen Feld einen Fehler haben kann. Das tatsächlich in dem Navigationsbereich herrschende elektromagnetische Feld weicht von dem ab, das von dem EM-Feldgenerator erzeugt wurde. Eine Haltevorrichtung der vorstehend beschriebenen Art weist eine Vielzahl an Bauteilen auf, die Einfluss auf das elektromagnetische Feld haben können, wie insbesondere elektromagnetische Bremsen in den Gelenken. Der Einfluss einer solchen Haltevorrichtung auf ein elektromagnetisches Feld ist daher vergleichsweise hoch. Basierend auf dem einen oder den mehreren 3D-Magnetometern kann gemäß dieser Ausführungsform die Pose der Haltevorrichtung relativ zu dem elektromagnetischen Feld, das in dem Navigationsbereich herrscht, bestimmt werden. Das Bestimmen der relativen Pose der Haltevorrichtung kann durch die Haltevorrichtung selbst, d.h. durch die Steuereinheit erfolgen. In einer Variante werden die von der Steuereinheit verarbeiteten Signale der 3D-Magnetometer drahtlos oder drahtgebunden an eine zum Haltearm externe Einheit, beispielsweise das OP-Navigationssystem, gesendet und diese bestimmt die relative Pose.

Das Vergleichen der relativen und der absoluten Posen der Haltevorrichtung kann ebenfalls entweder durch die Steuereinheit der Haltevorrichtung, oder durch eine Einheit, die extern zu der Haltevorrichtung ist, erfolgen. Basierend auf dem Vergleich wird dann ein Fehler, mithilfe einer Fehlerkorrektureinheit, eines elektromagnetischen Felds bestimmt. Die Fehlerkorrektureinheit kann eine Einheit der Steuereinheit der Haltevorrichtung sein, oder eine Einheit, die extern zu der Haltevorrichtung ist, beispielsweise in dem OP-Navigationssystem vorgesehen.

In einer bevorzugten Weiterbildung wird dieser bestimmte Fehler dazu verwendet, eine Position von einem im elektromagnetischen Feld befindlichen Objekt zu bestimmen. Das OP-Navigationssystem bestimmt die Position von einem oder mehreren in dem elektromagnetischen Feld befindlichen Objekten, wie insbesondere chirurgischen Instrumenten und dergleichen. Das das elektromagnetische Feld, das im Navigationsbereich herrscht, nicht identisch ist mit dem, das von dem EM-Feldgenerator erzeugt wurde, da es aufgrund, beispielsweise der Haltevorrichtung verändert ist, kann es vorkommen, dass die Position, die von dem OP-Navigationssystem bezüglich des Objekts bestimmt wurde, nicht korrekt ist. Da aber der Fehler des elektromagnetischen Felds bekannt ist, ist es möglich, die bezüglich des Objekts bestimmten Positionen zu korrigieren. Hierdurch ist die Genauigkeit der Positionsbestimmung von Objekten im Navigationsbereich verbessert.

In einer bevorzugten Weiterbildung umfasst der Schritt Bestimmen der absoluten Pose der Haltevorrichtung, Bestimmen von Stellungen der Gelenke der Haltevorrichtung; und Berechnen der absoluten Pose basierend auf den Stellungen der Gelenke. Dazu sind vorzugsweise Positionssensoren in den Gelenken vorgesehen, die über das interne BUSSystem mit der Steuereinheit verbunden sind. Durch die Erfassung der Stellung der einzelnen Gelenke, insbesondere deren Drehpositionen, kann die Pose der Haltevorrichtung berechnet werden. Die von der Steuereinheit, oder einer externen Einheit, berechnete Pose wird vorzugsweise an der proximalen Schnittstelle bereitgestellt, und an die Fehlerkorrektureinheit gesendet.

In einer Alternative oder zusätzlich, wird die absolute Pose der Haltevorrichtung bestimmt durch optisches Erfassen der absoluten Pose der Haltevorrichtung mittels einer optischen Erfassungseinheit. Das optische Erfassen basiert insbesondere auf IR-Reflexion. Das OP-Navigationssystem kann zusätzlich IR-Kameras aufweisen sowie IR-Sender die eine IR-Strahlung aussenden. An der Haltevorrichtung können IR-Reflektoren vorgesehen sein, sodass das OP-Navigationssystem basierend auf der gesendeten IR-Strahlung und der Reflexion die absolute Pose der Haltevorrichtung optisch bestimmen kann. Zusätzlich kann auch vorgesehen sein, dass die Haltevorrichtung selbst aktive IR-Sender aufweist, wobei das OP-Navigationssystem dazu vorgesehen ist, optisch basierend auf den von den aktiven IR-Sendern gesendeten Signalen, die absolute Pose der Haltevorrichtung zu bestimmen. Die Bezeichnung absolute Pose bezieht sich hier auf eine Pose unabhängig von dem elektromagnetischen Feld, insbesondere umfasst der Begriff absolute Pose auch eine Pose, die relativ zu einem OP-Tisch oder zum OP-Navigationssystem bestimmt ist. Da die Positionen des OP-Tisches und auch des OP-Navigationssystems bekannt sind, wird auch eine solche Pose als absolute Pose bezeichnet.

In einem fünften Aspekt wird ein Verfahren zur Kommunikation zwischen einer Haltevorrichtung oder einer Haltevorrichtung mit daran angeordnetem robotischen Anbaugerät und einem OP-Navigationssystem, vorgeschlagen mit den Schritten: Erfassen mittels einer an der Haltevorrichtung angeordneten Navigationskamera einen durch die Haltevorrichtung für das OP-Navigationssystem abgeschatteten Bereich; Bereitstellen von durch die Navigationskamera erfassten optischen Daten an dem OP-Navigationssystem. Die Navigationskamera kann, wie dies im ersten Aspekt der Erfindung vorgeschlagen wird, direkt an der Haltevorrichtung angeordnet sein, oder wie dies im dritten Aspekt der Erfindung vorgeschlagen wird mittelbar an der Haltevorrichtung angeordnet sein, nämlich direkt an dem robotischen Anbaugerät, welches seinerseits an der Haltevorrichtung befestigt ist.

Vorzugsweise umfasst das Verfahren den Schritt: Zusammenfügen von durch das OP-Navigationssystem erfassten optischen Daten und den durch die Navigationskamera erfassten optischen Daten zum Erhalten eines Bildes ohne einen durch die Haltevorrichtung abgeschatteten Bereich.

In einem sechsten Aspekt der Erfindung wird die eingangs genannte Aufgabe durch ein Computerprogramm nach Anspruch 15 gelöst mit Programmcode-Mitteln, die einen Prozessor einer Haltevorrichtung nach einer der vorstehend beschriebenen bevorzugten Ausführungsformen einer Haltevorrichtung gemäß dem ersten Aspekt der Erfindung zur Ausführung der Schritte des Verfahrens zur Erzeugung eines Codes nach einer der vorstehend beschriebenen Ausführungsformen eines Verfahrens gemäß dem vierten Aspekt der Erfindung veranlassen, wenn diese Programmcode-Mittel von dem Prozessor ausgeführt werden.

Nachstehend wird die Erfindung unter Bezugnahme auf die beiliegenden Figuren näher erläutert. Dabei zeigen:
- Fig. 1: eine Seitenansicht einer Haltevorrichtung gemäß der Erfindung;
- Fig. 2: eine weitere Ausführungsform der Haltevorrichtung gemäß der Erfindung;
- Fig. 3: eine weitere Ausführungsform der Haltevorrichtung gemäß der Erfindung;
- Fig. 4: ein schematisches Diagramm zum Aufbau der Haltevorrichtung gemäß einem weiteren Ausführungsbeispiel;
- Fig. 5: ein weiteres Diagramm eines Aufbaus einer Haltevorrichtung gemäß der Erfindung;
- Fig. 6: eine perspektivische Darstellung der Haltevorrichtung gemäß der Erfindung;
- Figuren 7a-7c: eine schematische Darstellung einer Anzeigeeinheit in drei verschiedenen Zuständen;
- Figuren 8a-8c: eine weitere schematische Darstellung einer Anzeigeeinheit in drei verschiedenen Zuständen;
- Figur 9: eine perspektivische Darstellung einer Haltevorrichtung mit daran aufgenommenem robotischen Anbaugerät in einem weiteren Ausführungsbeispiel;
- Figur 10: eine vergrößerte Darstellung des robotischen Anbaugeräts gemäß Figur 9;
- Figur 11: eine weitere perspektivische Darstellung der Haltevorrichtung samt robotischem Anbaugerät aus Figur 9 mit chirurgischem Instrument; und
- Figur 12: eine perspektivische Darstellung einer Haltevorrichtung gemäß einem weiteren Ausführungsbeispiel.

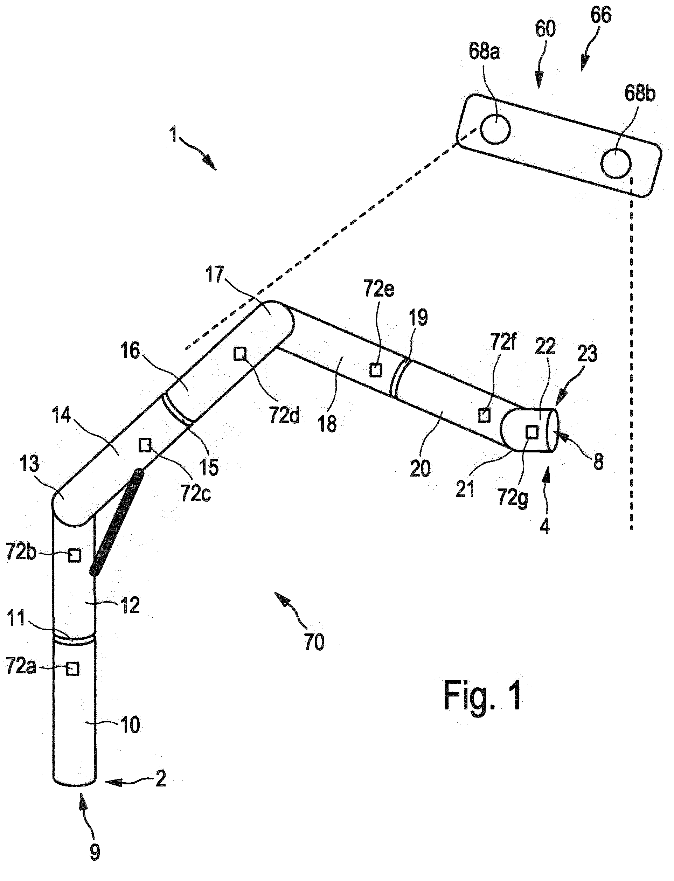

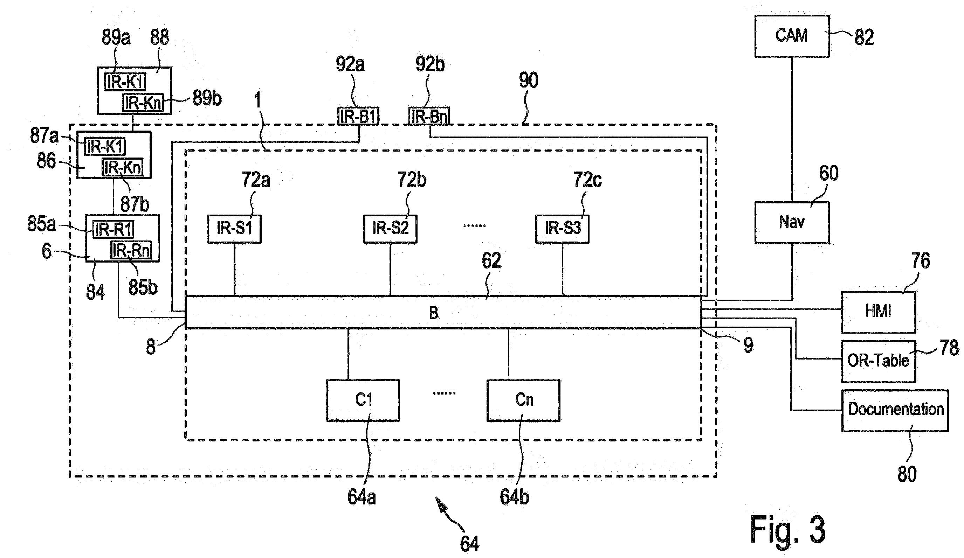

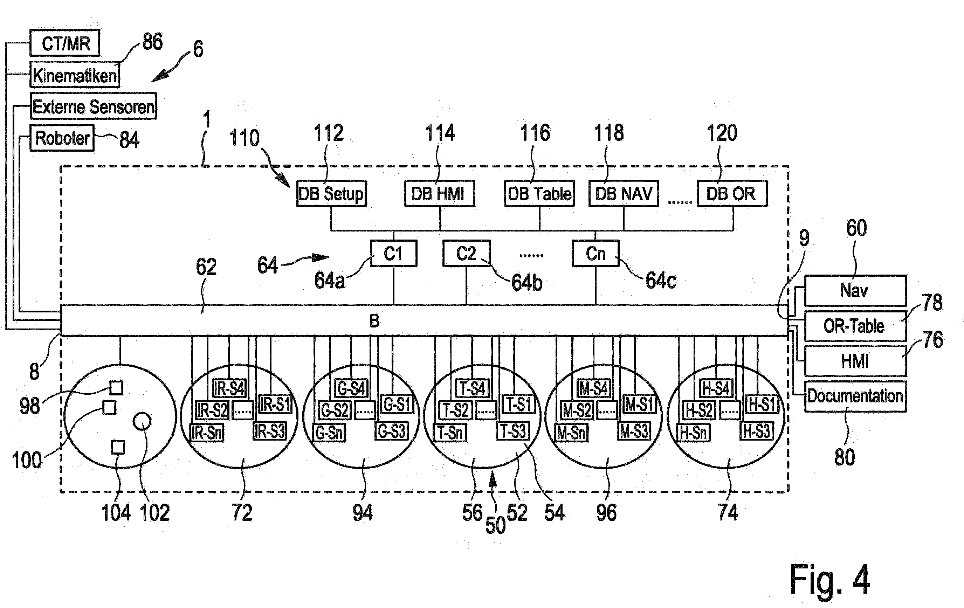

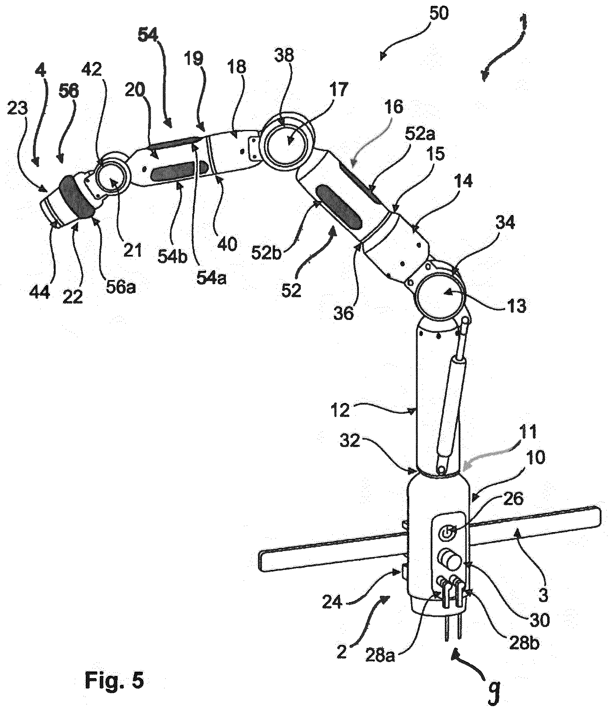

Eine Haltevorrichtung 1 (Fig. 1) hat die Form eines Haltearms und weist ein proximales Ende 2 zur Befestigung der Haltevorrichtung 1 an einer Basis 3 (vgl. Fig. 5) auf. Die Haltevorrichtung 1 weist ferner ein distales Ende 4 zum Aufnehmen eines Anbaugeräts 6 (vgl. Figuren 3-6) auf. Am distalen Ende 4 ist eine Schnittstelle 8 vorgesehen, die einerseits zum Koppeln mit dem Anbaugerät dient, andererseits auch zur Übertragung von Daten und elektrischer Energie an und von diesem. Eine zweite Schnittstelle 9 ist an dem proximalen Ende 2 vorgesehen und dient zum Anschluss der Haltevorrichtung 1 an ein OP-Navigationssystem 60 (vgl. insbesondere auch Figuren 3 und 4).

Insgesamt weist die Haltevorrichtung 1 gemäß diesem Ausführungsbeispiel sieben Armsegmente 10, 12, 14, 16, 18, 20, 22 auf, wobei zwischen den einzelnen Armsegmenten 10 bis 22 die Gelenke 11, 13, 15, 17, 19, 21, 23 vorgesehen sind. Die Gelenke 11, 15, 19 und 23 sind als rotatorische Gelenke ausgebildet und die Gelenke 13, 17 und 21 als Schwenkgelenke. Das heißt, bezogen auf Fig. 1, liegen die Drehachsen der Gelenke 11, 15, 19 und 23 im Wesentlichen innerhalb der Zeichenebene, während sich die Drehachsen der Gelenke 13, 17 und 21 im Wesentlichen senkrecht zur Zeichenebene erstrecken.

Die Haltevorrichtung 1 weist ferner ein BUS-System 62 auf, welches von der proximalen Schnittstelle 9 zur distalen Schnittstelle 8 verläuft und dort mit den entsprechenden Schnittstellen 8, 9 gekoppelt ist. Ferner weist die Haltevorrichtung 1 in jedem Armsegment eine Prozessoreinheit 64a, 64b auf (in Fig. 3 nur zwei gezeigt), die ebenfalls mit dem BUS-System 62 verbunden sind. Die jeweiligen Prozessoreinheiten 64a, 64b, die in jedem Armsegment vorgesehen sind (also insgesamt sieben im Ausführungsbeispiel aus Fig. 1) dienen dazu, einzelne Bremsen an den Gelenken 11 bis 23 zu steuern. Insgesamt bilden die Prozessoreinheiten 64a, 64b gemeinsam eine Steuereinheit 64 der Haltevorrichtung 1, die insgesamt Funktionen der Haltevorrichtung 1 steuert.

Die Haltevorrichtung 1 weist gemäß der Erfindung einen Empfänger 70 auf, der dazu vorgesehen ist, elektromagnetische Strahlung des OP-Navigationssystems zu empfangen, und der mit der Steuereinheit 64 verbunden ist und dazu angepasst ist, basierend auf den empfangenen elektromagnetischen Signalen des OP-Navigationssystems 60 Signale an die Steuereinheit 64 zu senden. Der Empfänger 70 weist gemäß dem Ausführungsbeispiel, welches in Fig. 1 dargestellt ist, eine Mehrzahl an Infrarot-Sensoren, nämlich IR-Fotodioden 72a, 72b, 72c, 72d, 72e, 72f, 72g (insgesamt als 72 bezeichnet; vgl. auch Fig. 3) auf. In Fig. 1 ist an jedem Armsegment 10 bis 22 jeweils eine IR-Fotodiode 72 angeordnet. Allerdings ist bevorzugt, dass zwei, drei, vier oder mehr IR-Fotodioden um den Umfang eines jeden Armsegments 10 bis 22 angeordnet sind, sodass unabhängig von der Pose der Haltevorrichtung 1 stets wenigstens eine IR-Fotodiode 72 so ausgerichtet ist, dass sie IR-Strahlung von dem OP-Navigationssystem 60 empfangen kann. Das OP-Navigationssystem 60 ist gemäß diesem Ausführungsbeispiel (Fig. 1) als optisches OP-Navigationssystem 66 ausgebildet, welches zwei Infrarot-Kameras 68a, 68b aufweist. Das OP-Navigationssystem 66 sendet Infrarot-Strahlung aus, beispielsweise in Form von IR-Blitzen oder Pulsen und kann hiermit der Haltevorrichtung 1 Befehle oder andere Informationen und Daten übertragen. Ferner ist die Haltevorrichtung 1 dazu ausgebildet, zu erkennen, dass sie sich in einer navigierten Umgebung befindet, wenn sie Infrarot-Strahlung von dem OP-Navigationssystem 66 mittels der IR-Fotodioden 72 empfängt. Die Steuereinheit 64 ist dann dazu ausgebildet, die Haltevorrichtung in einen Navigationsmodus zu versetzen, in dem einzelne Funktionen der Haltevorrichtung 1 geändert sind, insbesondere gesperrt sind.

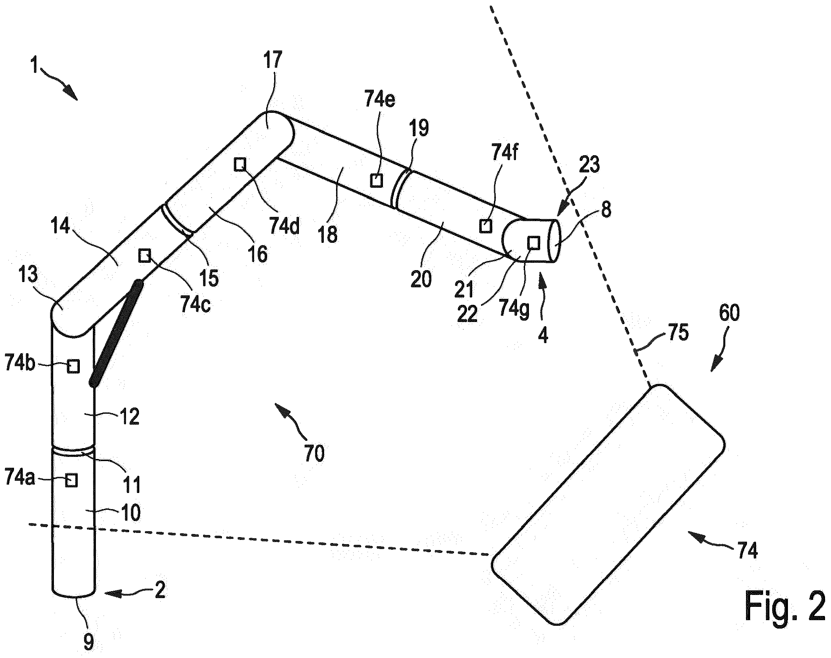

In Fig. 2 ist eine Variante zu der Ausführungsform gem. Fig. 1 dargestellt. Grundsätzlich ist auch in Fig. 2 eine Haltevorrichtung 1 gezeigt, die im Wesentlichen identisch zu der Haltevorrichtung gemäß Fig. 1 ausgebildet ist. Gleiche und ähnliche Elemente sind mit gleichen Bezugszeichen versehen und insofern wird vollumfänglich auf die obige Beschreibung zu Fig. 1 Bezug genommen. In dem Ausführungsbeispiel gemäß Fig. 2 ist das OP-Navigationssystem 60 so ausgebildet, dass es mit elektromagnetischer Strahlung arbeitet. Es weist daher einen EM-Feldgenerator 74 auf, der ein elektromagnetisches Feld 75 erzeugt. Üblicherweise sind mehrere solcher EM-Feldgeneratoren 74 vorgesehen. Zur Kommunikation mit dem OP-Navigationssystem 60 weist daher die Haltevorrichtung 1 gemäß diesem Ausführungsbeispiel (Fig. 2) wiederum einen Empfänger 70 auf, der gemäß diesem Ausführungsbeispiel eine Mehrzahl an Hall-Sensoren 74a, 74b, 74c, 74d, 74e, 74f, 74g aufweist (insgesamt mit 74 bezeichnet). In jedem Armsegment 10 bis 22 ist ein solcher Hall-Sensor 74 vorgesehen. Jeder Hall-Sensor 74 ist über das BUSSystem 62 mit der entsprechenden Prozessoreinheit 74a-c der Steuereinheit 64 verbunden, sodass jedes Armsegment 10 bis 22 selbstständig bestimmen kann, ob es sich im navigierten Feld befindet. Gemäß Fig. 2 sind alle Armsegmente 10 bis 22 innerhalb des elektromagnetischen Felds 75, wobei von dem Armsegment 10 nur die Hälfte innerhalb des Feldes ist.

Bezüglich der Hall-Sensoren 74 gilt im Wesentlichen dasselbe wie zu den IR-Fotodioden 72 beschrieben und die Haltevorrichtung 1 ist dazu vorgesehen, basierend auf von den Hall-Sensoren 74 empfangenen Signalen zu bestimmen, ob sie sich in einem navigierten Umfeld befindet, und entsprechend in einen Navigationsmodus zu wechseln.

Figuren 3 und 4 zeigen nochmals schematisch den Aufbau der Haltevorrichtung 1 sowie die Peripherie, wobei Fig. 3 im Wesentlichen dem Ausführungsbeispiel aus Fig. 1 entspricht und die Haltevorrichtung gemäß Fig. 4 zusätzlich weitere Elemente aufweist.

In Fig. 3 ist die Haltevorrichtung 1 gestrichelt dargestellt, und in deren Inneren verläuft das BUS-System 62 von der proximalen Schnittstelle 9 zur distalen Schnittstelle 8. Das BUS-System 62 ist mit den einzelnen Prozessoreinheiten 64a, 64b der Steuereinheit 64 sowie den IR-Fotodioden 72a, 72b, 72c gekoppelt. An der proximalen Schnittstelle 9 ist die Haltevorrichtung 1 kabelgebunden mit einem Server des OP-Navigationssystems 60 verbunden, um auf diese Weise Daten zu empfangen, oder weiterzugeben. Über die proximale Schnittstelle 9 ist die Haltevorrichtung 1 ferner an ein Fußpedal 76 gekoppelt, über welches alle Gelenke 11-23 freigebbar sind, sodass die Pose der Haltevorrichtung 1 insgesamt verstellt werden kann. Ferner ist die Haltevorrichtung 1 über die Schnittstelle 9 an den OP-Tisch 78 gekoppelt und auch mit einem Dokumentationssystem 80 verbunden. Das Dokumentationssystem 80 dient zur Dokumentation einer Operation, die in dem OP durchgeführt wird. Das Navigationssystem 60 seinerseits ist mit einem Computer 82 verbunden und empfängt von diesem CAM-Daten, die auch zur Ausrichtung der Haltevorrichtung 1 dienen.

Die Haltevorrichtung 1 ist an der distalen Schnittstelle 8 mit einem Anbaugerät 6 gekoppelt, welches hier als Robotereinheit 84 ausgebildet ist. Die Robotereinheit 84 weist ein oder mehrere Aktuatoren auf, die dazu dienen, eine an die Robotereinheit 84 gekoppelte Kinematik 86 zu betätigen. Die Kinematik 86 ihrerseits nimmt ein medizinisches Instrument 88 auf, wie beispielsweise ein Endoskop, eine Biopsienadel, oder dergleichen. Um die Haltevorrichtung 1 sowie die Robotereinheit 84 und die Kinematik 86 ist eine sterile Tüte 90 angeordnet. Zur verbesserten Kommunikation zwischen der Haltevorrichtung 1 und dem OP-Navigationssystem 60 ist auch die sterile Tüte 90 mit entsprechenden IR-Fotodioden 92a, 92b ausgestattet, die jeweils mit den Schnittstellen 8, 9 verbunden sind. Auch die Robotereinheit 84 weist zwei IR-Fotodioden 85a, 85b auf. Das Gleiche gilt in diesem Ausführungsbeispiel auch für die Kinematik 86, die zwei IR-Fotodioden 87a, 87b aufweist sowie auch für das Instrument 88, welches zwei IR-Fotodioden 89a, 89b aufweist. Auf diese Weise können auch die einzelnen Anbaugeräte 6, 86, 88 ihrerseits Daten und Informationen von dem OP-Navigationssystem 60 empfangen und entsprechend verarbeiten. Sofern die Kinematik 86 beispielsweise keine eigene Steuereinheit aufweist, werden die Daten an das BUS-System 62 weitergeleitet, über das diese dann mittels der Steuereinheit 64 verarbeitet werden können.

Gemäß Fig. 4 ist wiederum die Haltevorrichtung 1 gezeigt, wobei zur Vereinfachung die sterile Tüte 90 weggelassen ist. Gleiche Elemente sind mit gleichen Bezugszeichen versehen, sodass wiederum auf die obige Beschreibung zur Fig. 3 verwiesen wird.

Die Haltevorrichtung 1, in Fig. 4 dargestellt, weist neben den bereits mit Bezug auf Fig. 3 beschriebenen Elementen Winkelsensoren, insgesamt mit 94 bezeichnet, auf, die in jedem Gelenk 11 bis 23 angeordnet sind und eine Drehstellung des jeweiligen Gelenks 11 bis 23 messen. Die Winkelsensoren 94 sind über das BUS-System 62 mit der Steuereinheit 64 verbunden. Ferner weist die Haltevorrichtung 1 3D-Magnetometer 96 auf, wiederum jeweils ein 3D-Magnetometer in jedem Armsegment 10 bis 22 angeordnet, sodass basierend auf den Daten der Winkelsensoren 94 und den 3D-Magnetometern 96 die Pose der Haltevorrichtung 1 bestimmbar ist. Die Bestimmung der Pose der Haltevorrichtung 1 wird durch die Steuereinheit 64 vorgenommen. Die bestimmte Pose wird vorzugsweise über das BUS-System an die Schnittstelle 9 weitergegeben und von dort an das Navigationssystem 60.

Basierend auf der Pose, die mittels der Winkelsensoren 94 bestimmt wurde und basierend auf der Pose, die mittels der 3D-Magnetometer 96 bestimmt wurde, ist eine Abweichung dieser Posen, nämlich der absoluten und relativen Posen bestimmbar. Die Pose, die mittels der 3D-Magnetometer bestimmt wurde, weicht ab von der Pose, die mittels der Winkelsensoren 94 bestimmt wurde, da das elektromagnetische Feld einen Fehler aufweist. Durch den Vergleich dieser beiden Posen ist der Fehler bestimmbar. Basierend auf diesem Fehler kann eine Fehlerkorrektur auch bezüglich anderer in dem elektromagnetischen Feld befindlicher Objekte, wie insbesondere chirurgischer Geräte, vorgenommen werden. Hiermit wird die Genauigkeit der OP-Navigation wesentlich verbessert.

Weiterhin ist bevorzugt, dass die Haltevorrichtung 1 ein oder mehrere Beschleunigungssensoren 98, ein oder mehrere Gyroskope 100, ein Mikrofon 102 sowie ein oder mehrere Fotosensoren 104 aufweist. Auch die Beschleunigungssensoren 98, die Gyroskope 100, das Mikrofon 102 sowie die Fotosensoren 104 sind über das BUS-System mit der Steuereinheit 64 gekoppelt. Daher ist es unerheblich, in welchem der Armsegmente 10 bis 22 die einzelnen Sensoren angeordnet sind. Die Beschleunigungssensoren 98 sind vorzugsweise in jedem Segment 10 bis 22 vorgesehen, Gleiches gilt auch für die Gyroskope 100. Fotosensor und Mikrofon 102, 104 sind vorzugsweise im letzten Armsegment 22 angeordnet. Das Mikrofon dient insbesondere zur Sprachaufnahme und die Steuereinheit 64 weist eine entsprechende Spracherkennungssoftware auf, sodass mittels der Steuereinheit 64, Sprachbefehle einer Bedienperson in Stellsignale für die Haltevorrichtung 1, die Robotereinheit 84, und/oder die Kinematik 86 übersetzt werden können. Der oder die Fotosensoren 104 können dazu eingesetzt werden, eine Gestensteuerung für die Haltevorrichtung 1 vorzusehen, und/oder zu Dokumentationszwecken Screenshots einer OP-Situation vorzunehmen, und an das Dokumentationssystem 80 zu senden.

Wie sich ebenfalls aus der Fig. 4 ergibt, ist die Haltevorrichtung 1 mit einer Speichereinheit 110 ausgestattet, die dezentral, d.h. jeweils ein Teil in jedem Armsegment 10 bis 22, gebildet sein kann. Die Speichereinheit 110 ist mit der Steuereinheit 64 gekoppelt. Die Speichereinheit 110 weist mehrere Bereiche auf. In einem Bereich 112 sind vorzugsweise Konfigurationen der Haltevorrichtung 1, OP-Setups aus Gelenkwinkeln, angeschlossenen Geräten 6 usw. gespeichert. Beispielsweise sind hier bestimmte Posen, die für bestimmte Standardoperationen verwendet werden, vorgespeichert. Die Steuereinheit ist vorzugsweise dazu ausgebildet, eine bestimmte Pose der Haltevorrichtung mit einer der gespeicherten Posen in der Speichereinheit 112 zu vergleichen und ein entsprechendes Signal auszugeben, ob die gewünschte Pose (die gespeichert Pose) erreicht ist.

Ferner weist die Speichereinheit 110 einen Bereich 114 auf, in der Interaktionsmöglichkeiten und Benutzungsprofile für die Interaktion zwischen einem Bediener und der Haltevorrichtung 1 gespeichert sind. Hier ist beispielsweise gespeichert, ob in einem Navigationsmodus der Haltevorrichtung 1 die Haltevorrichtung 1 über eine manuelle Betätigung bewegt werden darf oder nicht. Auch sind hier beispielsweise für den Navigationsmodus und auch für den Bedienmodus verschiedene Maximalwerte für Winkelgeschwindigkeiten in Gelenken vorgesehen.

In einem weiteren Speicherbereich 116 sind Kommunikationsbefehle und interne Zustände gespeichert, die dann gelten, wenn die Haltevorrichtung 1 am Operationstisch angeschlossen ist. Beispielsweise können die Beschleunigungssensoren 98 in einem solchen Fall dazu verwendet werden, eine Bewegung des Operationstisches zu erfassen und entsprechend über das BUS-System 62 an die Steuereinheit 64, und/oder an das OP-Navigationssystem 60 auszugeben.

In einem weiteren Speicherbereich 118 sind Kommunikationsbefehle und interne Zustände gespeichert, die bei angeschlossener Navigation gelten. In diesem Speicherbereich 118 sind insbesondere die besonderen Bestimmungen für einen Navigationsmodus hinterlegt. Wenn bestimmt wird, dass sich die Haltevorrichtung 1 im navigierten Umfeld befindet, werden Profile aus diesem Speicherbereich 118 geladen. Weitere Speicherbereiche 120 können vorgesehen sein und für bestimmte Anwendungsfälle, bestimmte Profile und dergleichen speichern. Diese können ebenso durch einen Bediener vorgegeben werden, beispielsweise über ein Interface, was auf einem herkömmlichen PC gespeichert ist.

Gemäß Figur 5 ist eine Haltevorrichtung 1, in Form eines Haltearms, in einer Seitenansicht gezeigt. Gleiche uns ähnliche Elemente sind wiederum mit gleichen Bezugszeichen versehen und insofern wird vollumfänglich auf die obige Beschreibung verwiesen.

Die Haltevorrichtung weist an dem proximalen Ende 2 eine Basis 3 auf. Die Basis 3 ist gemäß diesem Ausführungsbeispiel als Normschiene eines Operationstisches ausgebildet (der Operationstisch ist in Figur 1 nicht gezeigt). Das erste Armsegment 10 bildet das proximale Ende 2 und weist eine Klemmklaue 24 auf, mittels der die Haltevorrichtung 1 an der Basis 3 festlegbar ist. An dem Armsegment 10 ist ferner ein Einschaltknopf 26 vorgesehen zum Einschalten der gesamten Haltevorrichtung 1, zwei Anschlüsse 28a, 28b der Schnittstelle 9 über die die Haltevorrichtung mit Strom und Daten, wie beispielsweise Stellsignalen und dergleichen, versorgbar ist, sowie ein Notausschalter 30.

Die Haltevorrichtung 1 weist an jedem Gelenk 11, 13, 15, 17, 19, 21, 23 eine Anzeigeeinheit 32, 34, 36, 38, 40, 42, 44 auf, die jeweils dazu vorgesehen sind, einen Status der Haltevorrichtung und/oder eines Anbaugeräts 6 (vergleiche Figur 2, 3 und 6) anzuzeigen.

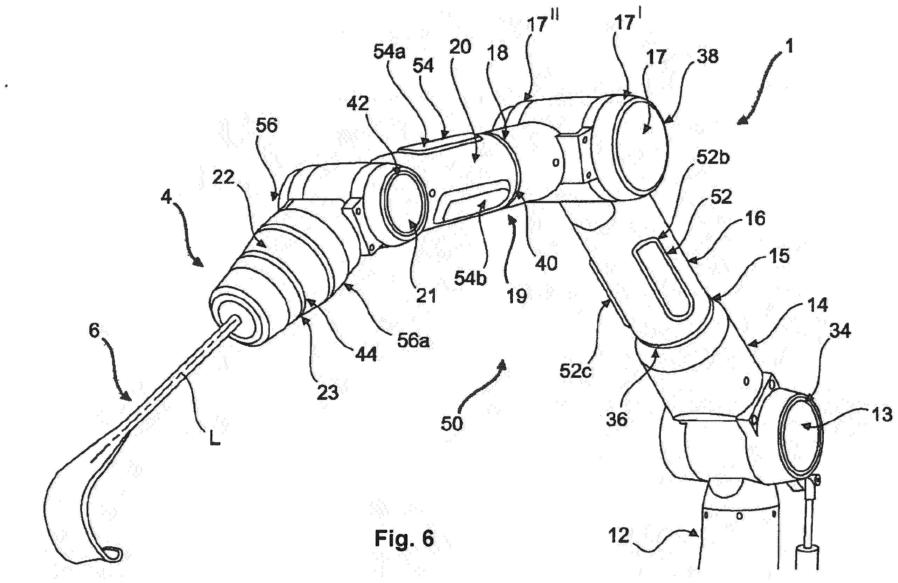

Die Anzeigeeinheiten 32, 34, 36, 38, 40, 42, 44 sind gemäß diesem Ausführungsbeispiel als im Wesentlichen ringförmig ausgebildet. Die Zentralachse von jedem Ring verläuft im Wesentlichen koaxial zur jeweiligen Drehachse des Gelenks 11, 13, 15, 17, 19, 21, 23. Während für die Gelenke 11, 15, 19, 23 jeweils ein einziger Ring vorgesehen ist, sind für die Gelenke 13, 17 und 21 jeweils zwei gegenüberliegende Ringe vorgesehen. Die zwei Ringe sind an vorderen und hinteren Gelenkabschnitten 17', 17" (in Figur 6 nur beispielsweise mit Bezugszeichen versehen) vorgesehen. So ist in jeder Lage der Haltevorrichtung 1 jede Anzeigeeinheit 32, 34, 36, 38, 40, 42, 44 stets erkennbar.

Gemäß diesem Ausführungsbeispiel (vergleiche Figuren 1 und 6) weist die Haltevorrichtung 1 ferner eine Bedieneinrichtung 50 auf. Mittels der Bedieneinrichtung 50 ist die Haltevorrichtung 1 in eine gewünschte Pose verbringbar, wobei die Bedieneinrichtung 50 dazu eingerichtet ist, bei Kontakt zwischen einer Bedienperson und einem der sieben Armsegmente 10, 12, 14, 16, 18, 20, 22, das zugeordnete Gelenk 11, 13, 15, 17, 19, 21, 23 freizugeben. Dazu weist die Bedieneinrichtung 28 gemäß diesem Ausführungsbeispiel drei Kontaktabschnitte 52, 54, 56 auf, wobei jeder Kontaktabschnitt 52, 54, 56 an einem anderen Armsegment 16, 20, 22 angeordnet ist. So ist am Armsegment 16 ein Kontaktabschnitt 52, am Armsegment 20 ein Kontaktabschnitt 54 und am Armsegment 22 ein Kontaktanschnitt 56 angeordnet. Jeder Kontaktabschnitt 52, 54, 56 weist separate Kontaktelemente 52a, 52b, 52c, 54a, 54b, 54c und 56a auf. Die einzelnen Kontaktelemente sind als berührungsempfindliche Oberflächen ausgebildet, sodass bei Kontakt zwischen einer Bedienperson und einem entsprechenden Kontaktmittel ein oder mehrere zugeordnete Gelenke freigegeben werden.

Gemäß diesem Ausführungsbeispiel sind an den Armsegmenten 16 und 20 jeweils drei Kontaktelemente 52a, 52b, 52c, 54a, 54b, 54c ausgebildet, an dem Armsegment 22 ist ein ringförmiges Kontaktelement 56 angeordnet, welches auch um seine Zentralachse drehbar ist, um so Funktionen an eine Schnittstelle am distalen Ende 4 aufgenommenen Anbaugeräts zu beeinflussen.

Die Zuordnung der einzelnen Gelenke 11, 13, 15, 17, 19, 21, 23 ist gemäß diesem Ausführungsbeispiel wie folgt geregelt: Bei Kontakt zwischen einer Bedienperson und dem Armsegment 16, also den Kontaktelementen 52a, 52b, 52c des Kontaktabschnitts 52 werden die Gelenke 15, 13 und 11 freigegeben. Eine Bedienperson kann nun drei Freiheitsgrade beeinflussen; dies ist ein Umfang, der manuell gut beherrschbar ist und in den manuell die Haltevorrichtung in eine gewünschte Pose bringbar ist. Kommt eine Bedienperson mit dem Armsegment 16 in Kontakt, und werden die Gelenke 15, 13 und 11 freigegeben, ist bevorzugt vorgesehen, dass die entsprechenden Anzeigeeinheiten 32, 34, 36 dieses Freigeben anzeigen, gemäß dem Ausführungsbeispiel der Figuren 1 und 2, also durch Aufleuchten des Rings.

Bei Kontakt zwischen dem Armsegment 20, also dem Kontaktabschnitt 54 und insbesondere den Kontaktmitteln 54a, 54b, 54c, werden die Gelenke 19 und 17 freigegeben. Entsprechend ist bevorzugt vorgesehen, dass die Anzeigeeinheiten 36, 38 dies anzeigen. Schließlich wird bei Kontakt zwischen dem Armsegment 22, also dem Kontaktabschnitt 56 und insbesondere dem Kontaktelement 56a die Gelenke 21 und 23 freigegeben, was bevorzugt mittels den Anzeigeeinheiten 42, 44 angezeigt wird.

Es soll verstanden werden, dass auch die Haltevorrichtung 1 der vorherigen Ausführungsbeispiele (Fig. 1 bis 4) eine solche Bedieneinrichtung 50 aufweisen können, auch wenn dies aus Übersichtlichkeitsgründen nicht gezeigt ist.

Nun bezugnehmend auf Figur 6 ist ein Anbaugerät 6 in Form eines Retraktors bzw. Wundspreizer an dem distalen Ende 4 aufgenommen. An der Schnittstelle am distalen Ende 4, an der der Retraktor 6 aufgenommen ist, sind eine oder mehrere Kraftsensoren angeordnet, mittels denen eine in Richtung der Längsachse L wirkende Zugkraft bestimmt werden kann. Ferner können mittels dieser Sensoren bevorzugt auch entsprechende Momente an der Schnittstelle um die Längsachse L, als auch senkrecht zu dieser, bestimmt werden. Die Anzeigeeinheit 44 ist dazu eingerichtet, diesen Status des Anbaugeräts 6 anzuzeigen, und insbesondere anzuzeigen, ob eine bestimmte Kraft innerhalb vorbestimmter Grenzen liegt. Bei Operationen besteht die Gefahr, dass ein Retraktor 6 über eine längere Zeit mit einer zu hohen Kraft beaufschlagt wird, wodurch das Gewebe, welches von dem Operationsfeld weggehalten wird, negativ beeinflusst wird. Durch Messen dieser Kraft und Bestimmung, ob diese innerhalb vorbestimmter Grenzen liegt, kann dieses Problem verringert bzw. vermieden werden.

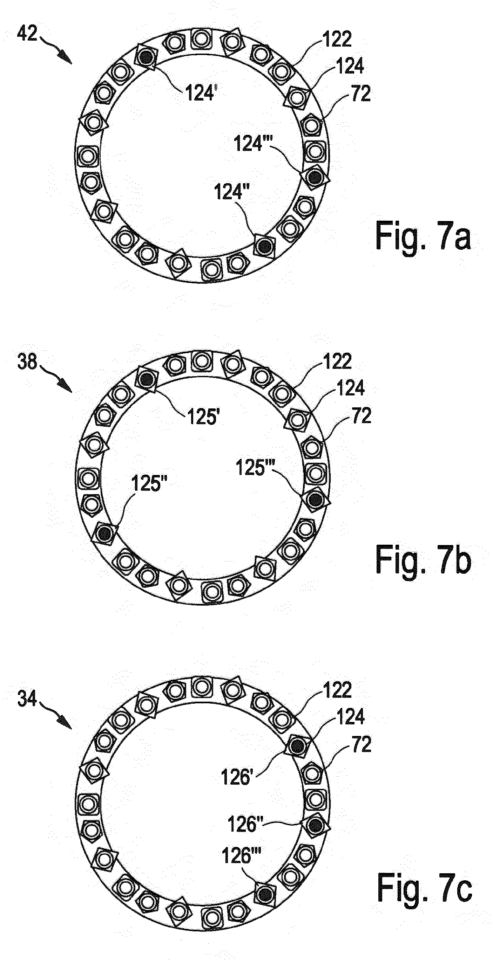

Die Figuren 7a bis 7c illustrieren, wie die Haltevorrichtung 1 über die Anzeigeeinheiten 32 bis 44 mit einem OP-Navigationssystem 60 kommunizieren kann, welches zum Empfang von Infrarotstrahlung ausgebildet ist. Die in den Fig. 7a bis 7c dargestellten Anzeigeeinheiten 34, 38, und 42 sind ringförmig ausgebildet und weisen eine Mehrzahl an Elementen auf, die in einem Ring angeordnet sind. Jeweils mit rechteckiger Umrandung dargestellt sind LEDs 122, die Licht im sichtbaren Bereich emittieren. Ein Element mit rautenförmiger Umrandung zeigt eine IR-LED 124 und ein Element mit fünfeckiger Umrandung eine IR-Fotodiode 72. Diese Elemente sind wechselseitig ringförmig angeordnet.

Durch die Dunkelfärbung von jeweils drei Elementen ist in den Figuren 7a bis 7c illustriert, dass bei der Anzeigeeinheit 42 drei IR-LEDs 124', 124", 124‴ aufleuchten, während die restlichen IR-LEDs 124 in Fig. 7a nicht leuchten. Damit bilden die drei IR-LEDs 124', 124", 124‴ in Fig. 7a ein eindeutiges Muster.

Das Gleiche gilt für die Anzeigeeinheit 38, die in Fig. 7b dargestellt ist. Dort leuchten die drei IR-LEDs 125', 125", 125‴ auf und bilden ebenfalls ein eindeutiges Muster, welches von dem Muster der IR-LEDs 124', 124", 124‴ aus Fig. 7a verschieden ist.

Auch die Anzeigeeinheit 34 weist LEDs 122, IR-LEDs 124 und IR-Fotodioden 72 auf. Bei der Anzeigeeinheit 34 gemäß Fig. 7c leuchten die drei IR-LEDs 126', 126", 126'" auf. Durch das Aufleuchten bilden sie wiederum ein eindeutiges Muster, welches von dem Muster der Anzeigeeinheiten 38 und 42 verschieden ist. Hierdurch ist es möglich, dass ein OP-Navigationssystem eindeutig die einzelnen Gelenke, an denen die Anzeigeeinheiten 34, 38, 42 vorgesehen sind, nämlich die Gelenke 13, 17 und 21 identifiziert und dadurch die Pose der Haltevorrichtung 1, basierend auf der IR-Emission, erkennen kann.

Es kann gleichzeitig vorgesehen sein, dass die jeweils benachbart zu einer entsprechenden IR-LED 124', 124", 124‴,125', 125", 125‴,126', 126", 126'" angeordnete LED, die Licht im sichtbaren Bereich emittiert, ebenfalls aufleuchtet. Hierdurch ist es für einen Bediener möglich zu erkennen, in welchem Muster die entsprechende Anzeigeeinheit 34, 38, 42 IR-Strahlung emittiert.

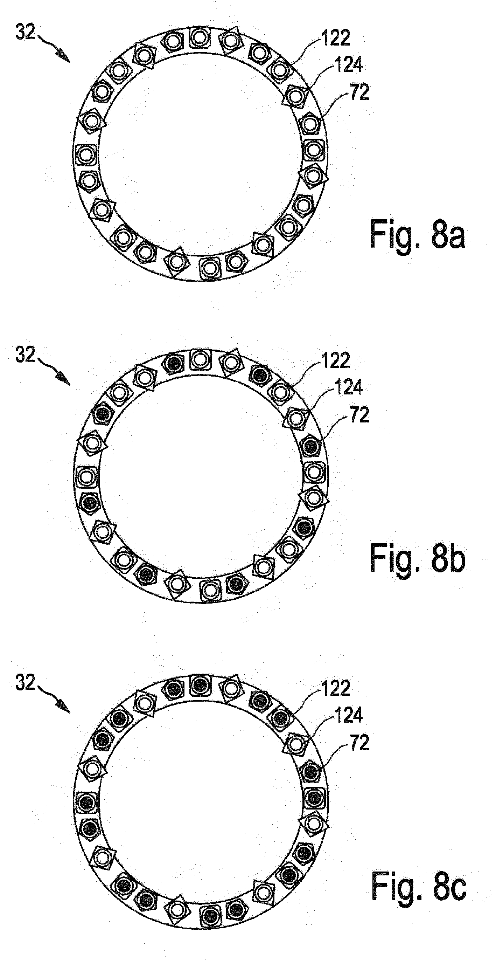

Die Figuren 8a bis 8c hingegen illustrieren jeweils dieselbe Anzeigeeinheit, einfacher Weise als 32 bezeichnet, in drei verschiedenen Zuständen. Wiederum sind die Elemente, die rechteckig umrandet sind, LEDs, die Licht im sichtbaren Wellenlängenbereich emittieren, die Elemente mit einer rautenförmigen Umrandung IR-LEDs und die Elemente mit fünfeckiger Umrandung IR-Fotodioden. In dem in Fig. 8a gezeigten Zustand ist keines der Elemente 122, 124, 72 aktiv. In Fig. 8b ist dargestellt, dass die Anzeigeeinheit 32 ein IR-Signal des OP-Navigationssystems 60 empfängt, insbesondere einen IR-Blitz. Die IR-Fotodioden 72 sind aktiv. Fig. 8c zeigt ein demgegenüber verändertes Ausführungsbeispiel, bei dem, wenn die IR-Fotodioden 72 ein Signal empfangen, gleichzeitig die LEDs 122, die Licht im sichtbaren Wellenbereich emittieren, aufleuchten. Alternativ kann vorgesehen sein, dass die LEDs 122, die Licht im sichtbaren Wellenlängenbereich emittieren, die Farbe wechseln. Hierdurch erhält der Bediener ein unmittelbares optisches Feedback darüber, ob die Haltevorrichtung 1 IR-Signale des OP-Navigationssystems empfängt. Hierdurch ist die Sicherheit weiter verbessert.

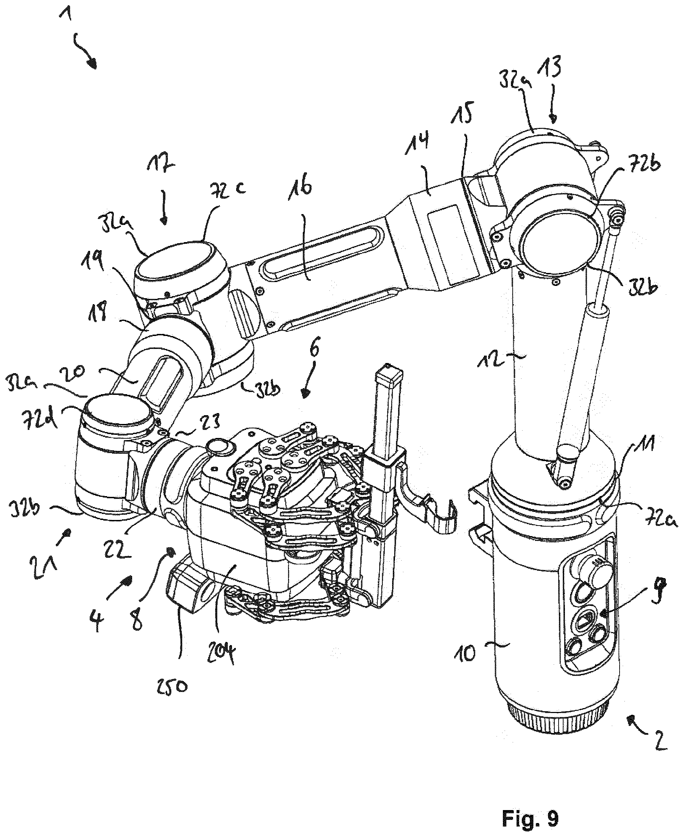

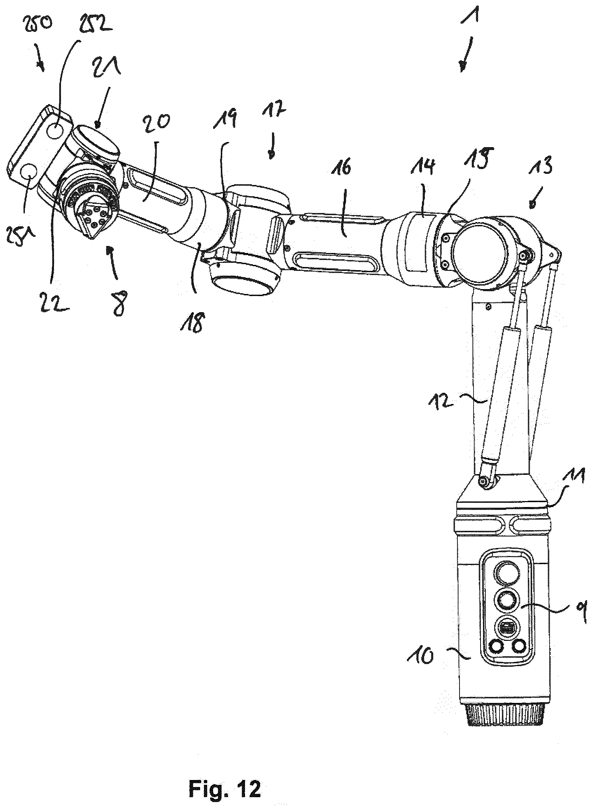

Die nachfolgenden Figuren 9 bis 12 zeigen weitere Ausführungsbeispiele der Haltevorrichtung samt einem daran angeordneten robotischen Anbaugerät (Figuren 9-11). Fig. 12 zeigt ein weiteres Ausführungsbeispiel, wobei in Fig. 12 das robotische Anbaugerät weggelassen ist, auch wenn dieses vorhanden sein kann.

Auch wenn in den Figuren 9 bis 12 das OP-Navigationssystem 60 nicht stets gezeigt ist, soll verstanden werden, dass dieses vorhanden ist und insofern wird Bezug genommen insbesondere auf Fig. 1.

Gleiche und ähnliche Elemente sind in den Ausführungsbeispielen gemäß Figuren 9 bis 12 mit denselben Bezugszeichen wie bei den ersten Ausführungsbeispielen versehen und insofern wird vollumfänglich auf die obige Beschreibung Bezug genommen. Im Nachfolgenden werden insbesondere die Unterschiede zu den ersten beiden Ausführungsbeispielen hervorgehoben.

Die Haltevorrichtung 1 weist gemäß Fig. 9 Armsegmente 10, 12, 14, 16, 18, 20, 22 sowie Gelenke 11, 13, 15, 17, 19, 21, 23 auf, wie mit Bezug auf die vorherigen Ausführungsformen bereits beschrieben.

Die Infrarot-Sensoren 72a, 72b, 72c, 72d sind in LED-Ringe integriert, wie dies bereits mit Bezug auf die Figuren 7a bis 8c beschrieben wurde. Diese Ringe sind insbesondere um die Schwenkachsen 13, 17 und 21 angeordnet. Dabei sind jeweils zwei solcher als Anzeigeeinheiten 32a, 32b ausgebildeten LED-Ringe an gegenüberliegenden Seiten der in etwa tonnenförmigen Gelenkkörper um die Schwenkgelenke 13, 17, 21 herum angeordnet. So sind die LED-Ringe der Anzeigeeinheiten 32a, 32b aus jeder Pose sichtbar und bilden eine Line-of-sight mit der Navigationskamera 68a, 68b des OP-Navigationssystems 66.

Das robotische Anbaugerät 6 ist abnehmbar an der distalen Schnittstelle 8 der Haltevorrichtung 1 angeordnet. Das robotische Anbaugerät 6 und insbesondere seine Kinematik sind beschrieben in der deutschen Patentanmeldung DE 10 2017 111 296, deren Offenbarungsgehalt hiermit vollumfänglich mit einbezogen wird.

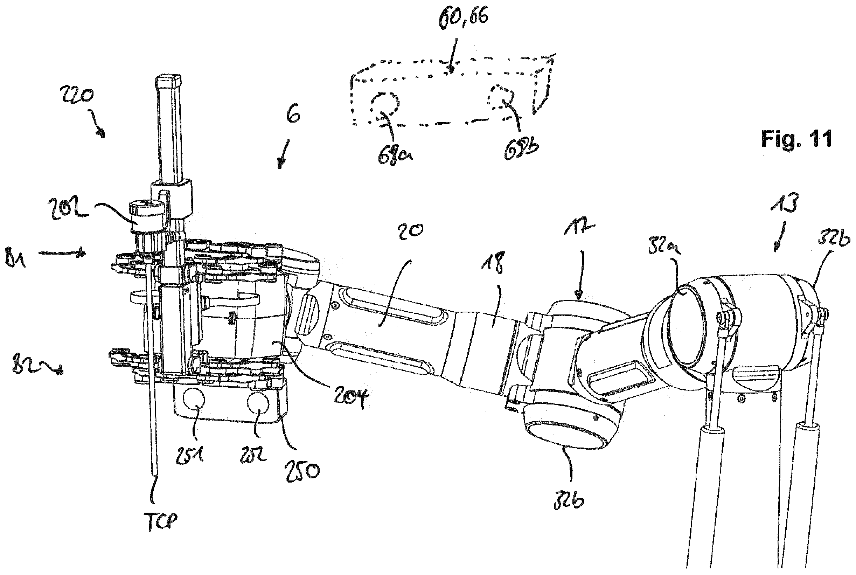

Das robotische Anbaugerät 6, welches hier als chirurgische Manipulatorvorrichtung ausgebildet ist, ist dazu vorgesehen, beispielsweise ein Endoskop 202 (vgl. Fig. 11) aufzunehmen. Das Anbaugerät 6 weist ein Gehäuse 204 auf, das eine Schnittstelle 206 (in Figuren nicht zu sehen, da sie mit der distalen Schnittstelle 8 verbunden ist) hat über die das Anbaugerät 6 mit der distalen Schnittstelle 8 der Haltevorrichtung 1 gekoppelt ist. Bezüglich der Schnittstelle wird daher auf die deutsche Patentanmeldung DE 10 2017 111 296 verwiesen.

Das Anbaugerät 6 weist darüber hinaus einen Rahmen im Inneren auf, der von dem Gehäuse 204 umgeben ist. Der Rahmen definiert die Struktur des Anbaugeräts 6. Der Rahmen ist in Figuren 9, 10, 11 und 12 nicht zu sehen, da er von dem Gehäuse 204 umgeben ist.

An dem Rahmen sind eine erste Lenkeranordnung 210 und eine zweite Lenkeranordnung 212 gelagert. Die erste Lenkeranordnung 210 ist in einer ersten Bewegungsebene bewegbar und die zweite Lenkeranordnung ist in einer zweiten Bewegungsebene bewegbar. Die Bewegungsebenen der Lenkeranordnung 210, 212 sind parallel zueinander und nicht gegeneinander verkippbar.

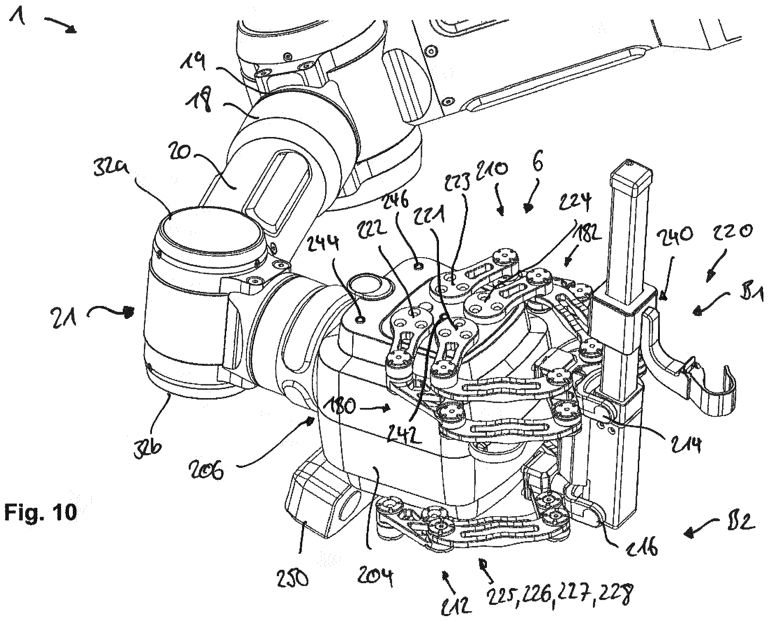

Die erste Lenkeranordnung 210 verbindet den Rahmen mit einem ersten Halter 214 und die zweite Lenkeranordnung 212 verbindet den Rahmen mit einem zweiten Halter 216. Mittels der Halter 214, 216 ist an dem Anbaugerät 6 eine Instrumentenaufnahmeeinrichtung 220 befestigt. Die erste Lenkeranordnung 210 ist an vier Hebelgelenkpunkten 221, 222, 223, 224 der ersten Lenkeranordnung 210 mit dem Rahmen gekoppelt, und die zweite Lenkeranordnung 212 ist an vier Hebelgelenkpunkten 225, 226, 227, 228 der zweiten Lenkeranordnung 212 mit dem Rahmen gekoppelt. Der erste Hebelgelenkpunkt 221 weist eine erste Rotationsachse, der zweite Hebelgelenkpunkt 222 eine zweite Rotationsachse, der dritte Hebelgelenkpunkt 223 eine dritte Rotationsachse und der vierte Hebelgelenkpunkt 224 eine vierte Rotationsachse auf. Die vier Hebelgelenkpunkte 225, 226, 227, 228 der zweiten Lenkeranordnung 212 sind als fünfter Hebelgelenkpunkt, sechster Hebelgelenkpunkt, siebter Hebelgelenkpunkt und achter Hebelgelenkpunkt bezeichnet. In diesem Ausführungsbeispiel weisen die vier Hebelgelenkpunkte 221, 222, 223, 224 der ersten Lenkeranordnung 210 gemeinsame Rotationsachsen mit den vier zweiten Hebelgelenkpunkten 225, 226, 227, 228 der zweiten Lenkeranordnung 212 auf. Insgesamt sind die ersten und zweiten Lenkeranordnungen 210, 212 spiegelsymmetrisch und identisch ausgebildet. Dies kann besonders gut aus Fig. 10 sowie 11 erkannt werden.

Die erste Lenkeranordnung 210 weist erste und zweite Armsegmente 180, 182 auf, die in sich wiederum identisch und spiegelsymmetrisch ausgebildet sind. Die zweite Lenkeranordnung 212 weist entsprechende erste und zweite Armsegmente auf, die wiederum in sich identisch und spiegelsymmetrisch ausgebildet sind, auch wenn diese nicht mit Bezugszeichen in den Figuren versehen sind.

Jedes der Armsegmente 180, 182 umfasst zwei Parallelogramme, nämlich ein erstes Parallelogramm, ein zweites Parallelogramm, ein drittes Parallelogramm, ein viertes Parallelogramm. Die zweite Hebelgelenkanordnung weist entsprechende Parallelogramme auf.

Die erste Lenkeranordnung 210 weist einen ersten Hebel, einen zweiten Hebel, einen dritten Hebel, und einen vierten Hebel auf, deren Drehachsen jeweils die Rotationsachsen sind. In entsprechender Weise weist die zweite Lenkeranordnung 212 einen fünften Hebel, einen sechsten Hebel, einen siebten Hebel und einen achten Hebel auf, deren Drehachsen ebenfalls die vier genannten Rotationsachsen sind. Sämtliche der Hebel sind abtriebsseitig mit einem Lenker verbunden. Die ersten und zweiten Hebel sind mit einem ersten Lenker verbunden, der dritte und vierte Hebel sind abtriebsseitig mit einem zweiten Lenker verbunden. Die fünften und sechsten Hebel der zweiten Lenkeranordnung sind abtriebsseitig mit einem dritten Lenker verbunden und die siebten und achten Hebel der zweiten Lenkeranordnung 212 sind abtriebsseitig mit einem vierten Lenker verbunden. So bilden der erste Lenker und der Rahmen gemeinsam ein erstes Parallelogramm. Die anderen Parallelogramme sind entsprechend gebildet.

Die erste Lenkeranordnung 210 weist ferner einen ersten Stab, einen zweiten Stab, einen dritten Stab und einen vierten Stab auf. Die zweite Lenkeranordnung weist entsprechende fünfte bis achte Stäbe auf. Der erste und zweite Stab verbindet den ersten Lenker gelenkig mit dem ersten Halter 214 und der dritte und vierte Stab verbinden den zweiten Lenker gelenkig mit dem ersten Halter 214. In entsprechender Weise verbinden auch die Stäbe der zweiten Lenkeranordnung 212 die Lenker mit dem zweiten Halter 216.

An den Haltern 214, 216 ist zudem ein Kardangelenk vorgesehen, um eine unterschiedliche Verschwenkung der Hebel und somit eine unterschiedliche Positionierung der Halter in den Bewegungsebenen B1, B2 zu ermöglichen.

Die Instrumentenaufnahme 220 weist zudem eine Linearführung 240 auf, die ein Bewegen des Instruments 202 senkrecht zu den Bewegungsebenen B1, B2 oder schräg zu diesen erlaubt.

Das Anbaugerät 206 weist in diesem Ausführungsbeispiel ferner einen Empfänger 242, insbesondere eine IR-Fotodiode auf, um elektromagnetische Strahlung des OP-Navigationssystem 60 zu empfangen. Der Empfänger 242 ist dazu mit einer Roboter-Steuereinheit, die innerhalb des Gehäuses 204 vorgesehen ist, verbunden. Der Empfänger sendet, basierend auf empfangenen elektromagnetischen Signalen des OP-Navigationssystems 60, Signale an die Roboter-Steuereinheit. Erkennt die Roboter-Steuereinheit dann, dass die empfangene elektromagnetische Strahlung von einem OP-Navigationssystem 60 gesendet wurde, schaltet die Roboter-Steuereinheit in einen Navigationsmodus des Anbaugeräts 6. In diesem Navigationsmodus kann beispielsweise vorgesehen sein, dass der Empfänger 242 permanent auf Signale wartet, oder dass bestimmte Bewegungen der ersten und zweiten Lenkeranordnung 210, 212 erlaubt und oder unterbunden sind. Im Navigationsmodus des Anbaugeräts 6 kann zudem vorgesehen sein, dass dieses mittels Sendern 244, 246 elektromagnetische Strahlung sendet, um einen bestimmten Status des Anbaugeräts 6, wie zum Beispiel die Pose oder dergleichen, mittels elektromagnetischer Strahlung drahtlos an das OP-Navigationssystem 60 zu übermitteln. Ferner können die Sender 244, 246 auch dazu verwendet werden, die Identität des Anbaugeräts 6 zu erkennen zu geben, sodass das OP-Navigationssystem 60 das Anbaugerät 6 wahrnehmen kann. So ist es insbesondere bei OP-Navigationssystemen 60, die mittels Navigationskameras und Infrarot-Strahlung arbeiten üblich, dass das OP-Navigationssystem nur Objekte erkennen kann, die selbst InfrarotStrahlung emittieren. Hierzu werden in der Regel sogenannte Tracker angebracht, die Infrarot-Strahlung, die von dem OP-Navigationssystem 60 ausgesandt wurde, reflektieren. Weist das Anbaugerät 6 hingegen aktive Sender 244, 246 auf, ist das Anbringen von zusätzlichen Trackern nicht erforderlich, da deren Funktionalität durch die Sender 244, 246 übernommen werden kann. Die Sender 244, 246 können dann beispielsweise auch blinken, um über dieses Blinken bestimmte Informationen an das OP-Navigationssystem 60 zu übertragen.

Weiterhin ist gemäß dieser Ausführungsform (Figuren 9-11) vorgesehen, dass das Anbaugerät 6 über eine Navigationskamera 250 verfügt. Die Navigationskamera 250 des Anbaugeräts 6 ist insbesondere in Fig. 11 gut zu sehen. Die Navigationskamera 250 ist am unteren Bereich des Anbaugeräts 6 angeordnet, wobei sich unten hier auf eine übliche Ausrichtung des Anbaugeräts 6 bezieht. Selbstverständlich ist es auch möglich, dass das Anbaugerät 6 eine andere Pose einnimmt und beispielsweise um seine eigene Achse gedreht ist.

Die Navigationskamera 250 weist erste und zweite Linsen 251, 252 auf, die auf das Operationsfeld, das heißt einen Tool-Center-Point TCP des chirurgischen Instruments 202 gerichtet sind. Es kann vorgesehen sein, dass die Navigationskamera 250 relativ zum Anbaugerät 6 schwenkbar ist und hierzu ein entsprechender Antrieb vorgesehen ist. So ist es beispielsweise denkbar und bevorzugt, dass bei einer Änderung der Position des chirurgischen Instruments 202 auch die Navigationskamera 250 verschenkt wird, um eine optimale Sicht auf das Operationsfeld zu erhalten. Die Navigationskamera 250 kann insbesondere den Bereich aufnehmen, der durch die Haltevorrichtung und/oder das Anbaugerät 6 in Bezug auf eine OP-Navigationskamera 68a, 68b eines OP-Navigationssystems 66 abgeschattet ist. Auch dies kann aus Fig. 11 anhand des nur schematisch dargestellten OP-Navigationssystems 66 erkannt werden.

Die Navigationskamera 250 kann also das Operationsfeld aufnehmen, auch wenn dieses durch die Haltevorrichtung 1 und/oder das Anbaugerät 6 gegenüber dem OP-Navigationssystem 60 abgeschattet ist. Die von der Navigationskamera 250 aufgenommenen Signale können zunächst an das Anbaugerät 6 weitergegeben werden, von dort über die Schnittstelle 206 und die proximale Schnittstelle 8 an die Haltevorrichtung 1, insbesondere an deren internes Bussystem, und dann über die proximale Schnittstelle 9 bereitgestellt werden. Dabei kann vorgesehen sein, dass das Anbaugerät 6 und/oder die Haltevorrichtung 1 die von der Navigationskamera 250 aufgenommenen Signale prozessieren. Ein solches Prozessieren kann insbesondere umfassen: Verknüpfen mit Daten, die die Position der Navigationskamera 250 angeben, Mitteilung über Instrumente, die sich im Operationsfeld befinden und dergleichen.

In einem weiteren Ausführungsbeispiel (Fig. 12) ist die Navigationskamera 250 an der Haltevorrichtung 1 angeordnet. In diesem Ausführungsbeispiel ist es nicht erforderlich, dass das Anbaugerät 6 mit einer Navigationskamera ausgestattet ist. Vielmehr kann unabhängig von dem an der Schnittstelle 8 aufgenommenen Anbaugerät 6 da OP-Feld durch die Navigationskamera 250, die direkt mit der Haltevorrichtung 1 verbunden ist, aufgenommen werden. Auch ist es möglich, die Navigationskamera 250 als zusätzliche Navigationskamera mittels der Haltevorrichtung 1 einzusetzen, ohne dass ein spezielles Anbaugerät 6 an der Schnittstelle 8 aufgenommen ist. Dies ist besonders dann vorteilhaft, wenn andere Geräte im OP-Feld angeordnet sind, die das OP-Feld teilweise gegenüber dem Navigationssystem 60 abschatten. Die Navigationskamera 250 kann in diesem Fall direkt mit dem internen Bussystem der Haltevorrichtung 1 gekoppelt sein. In dem mit Fig. 12 gezeigten Ausführungsbeispiel ist die Navigationskamera mit dem vorletzten Armsegment 20 verbunden und dort insbesondere im Bereich des Gelenks 21. Die Navigationskamera 250 kann aber auch an einem letzten Armsegment 22 angeordnet sein, was den Vorteil hat, dass hierdurch eine leichtere Einstellung der Orientierung der Navigationskamera 250 erreichbar ist.

## Patentansprüche

1. Haltevorrichtung (1), insbesondere Haltearm, für medizinische Zwecke zum Halten eines Anbaugeräts, insbesondere eines chirurgischen mechatronischen Assistenzsystems und/oder chirurgischen Instruments, mit
einem proximalen Ende (2) zum Befestigen der Haltevorrichtung (1) an einer Basis und einem distalen Ende (4) zum Aufnehmen eines Anbaugeräts (6);
wenigstens einem ersten und einem zweiten Armsegment (12, 14), wobei das erste Armsegment (12) mit einem ersten Gelenk (13) und das zweite Armsegment (14) mit einem zweiten Gelenk (15) verbunden ist, wobei jedes Gelenk (13, 15) freigebbar und arretierbar ist;
einer Bedieneinrichtung (50) zum Freigeben und/oder Arretieren des entsprechenden Gelenks (13, 15) zum Verbringen der Haltevorrichtung (1) in eine gewünschte Pose; und
einer Steuereinheit (64), umfassend einen Prozessor und Programmcodemittel, zum Steuern der Haltevorrichtung (1);
wenigstens einem Empfänger (70) für elektromagnetische Strahlung, der mit der Steuereinheit (64) verbunden ist und dazu angepasst ist, basierend auf empfangenen elektromagnetischen Signalen eines OP-Navigationssystems (60) Signale an die Steuereinheit zu (64) senden,
**dadurch gekennzeichnet, dass** die Haltevorrichtung (1) dazu eingerichtet ist, dass sie in einem Navigationsmodus und einem Bedienmodus betreibbar ist,
wobei die Haltevorrichtung (1) in dem Bedienmodus rein manuell über eine für die Haltevorrichtung (1) vorgesehene Bedieneinheit (50) bedienbar ist, und in dem Navigationsmodus dazu vorbereitet und eingerichtet ist, Befehle von dem Navigationssystem zu empfangen und von diesem gesteuert zu werden,
wobei die Steuereinheit (64) dazu eingerichtet ist, sich basierend auf den von dem Empfänger an die Steuereinheit gesendeten Signalen zu bestimmen, ob sich die Haltevorrichtung (1) in einer navigierten OP-Umgebung befindet oder nicht in einer navigierten OP-Umgebung befindet,
und wobei die Haltevorrichtung (1) automatisch in den Bedienmodus schaltet, falls sie sich nicht in einer navigierten OP-Umgebung befindet, und automatisch in den Navigationsmodus schaltet, falls sie sich in einer navigierten OP-Umgebung befindet.

2. Haltevorrichtung (1) nach Anspruch 1, ferner aufweisend wenigstens einen Sender zum Senden elektromagnetischer Strahlung zum Senden von Signalen an ein OP-Navigationssystem (60), wobei der Sender vorzugsweise eine IR-Lichtquelle (124) aufweist.

3. Haltevorrichtung (1) nach Anspruch 2, aufweisend eine erste Anzeigeeinheit (34), die an dem ersten Gelenk (13) angeordnet ist und eine zweite Anzeigeeinheit (36), die an dem zweiten Gelenk (15) angeordnet ist, wobei die erste und/oder zweite Anzeigeeinheit (34, 36) jeweils wenigstes eine IR-Lichtquelle (124) aufweisen und dazu eingerichtet sind, wenigstens einen Status der Haltevorrichtung (1) und/oder einen Status eines Anbaugeräts (6) anzuzeigen.

4. Haltevorrichtung (1) nach Anspruch 2 oder 3, wobei der eine oder die mehreren Sender dazu ausgebildet ist/sind, ein Signal auszugeben, wenn eine oder mehrere Bremsen an den Gelenken (11, 13, 15, 17, 19, 21, 23) geöffnet werden und/oder ein oder mehrere Gelenke (11, 13, 15, 17, 19, 21, 23) der Haltevorrichtung (1) bewegt werden.

5. Haltevorrichtung (1) nach einem der vorstehenden Ansprüche, ferner aufweisend eine Navigationskamera zum Erfassen eines Operationsfelds, wobei die Haltevorrichtung dazu eingerichtet ist, von der Navigationskamera erfasste Signale prozessiert oder unprozessiert an einer Schnittstelle für das OP-Navigationssystem bereitzustellen.

6. Haltevorrichtung (1) nach einem der vorstehenden Ansprüche, ferner aufweisend ein BUS-System sowie eine erste mechatronische Schnittstelle an dem proximalen Ende und eine zweite mechatronische Schnittstelle an dem distalen Ende, wobei die mechatronische Schnittstelle an dem distalen Ende zum Koppeln mit einem robotischen Anbaugerät vorgesehen ist.

7. Robotisches Anbaugerät (6), insbesondere Manipulatorvorrichtung, mit:
einem Rahmen,
einem an dem Rahmen abgestützten Antrieb,
einer Instrumentenaufnahme (220) zum Halten eines chirurgischen Instruments (202), die mittels des Antriebs antreibbar ist, und
einer mechatronischen Schnittstelle (206) zum Koppeln mit einer distalen Schnittstelle (8) einer Haltevorrichtung (1) nach einem der vorstehenden Ansprüche,
**gekennzeichnet durch** wenigstens einen Empfänger (242) für elektromagnetische Strahlung, der mit einer Roboter-Steuereinheit verbunden ist und dazu angepasst ist, basierend auf empfangenen elektromagnetischen Signalen eines OP-Navigationssystems (60) Signale an die Roboter-Steuereinheit zu senden,
wobei das robotische Anbaugerät (6) in einem Roboter-Navigationsmodus und einem Roboter-Bedienmodus betreibbar ist, wobei das robotische Anbaugerät (6) in dem Roboter-Bedienmodus rein manuell über eine für das Anbaugerät (6) vorgesehene Roboter-Bedieneinheit bedienbar ist, und in dem Roboter-Navigationsmodus dazu vorbereitet ist, Befehle von dem Navigationssystem (60) zu empfangen,
wobei die Roboter-Steuereinheit dazu eingerichtet ist basierend auf den von dem Empfänger (242) an die Roboter-Steuereinheit gesendeten Signalen zu bestimmen, ob sich das Anbaugerät (6) in einer navigierten OP-Umgebung befindet oder nicht in einer navigierten OP-Umgebung befindet, und wobei das Anbaugerät (6) automatisch in den Roboter-Bedienmodus schaltet, falls es sich nicht in einer navigierten OP-Umgebung befindet und automatisch in den Roboter-Navigationsmodus schaltet, falls es sich in einer navigierten OP-Umgebung befindet.

8. Robotisches Anbaugerät nach Anspruch 7, **gekennzeichnet durch** eine Navigationskamera (250) zum Erfassen eines Operationsfelds, wobei das robotische Anbaugerät (6) dazu eingerichtet ist, von der Navigationskamera (250) erfasste Signale prozessiert oder unprozessiert an einer Schnittstelle für das OP-Navigationssystem und/oder der mechatronischen Schnittstelle (206) bereitzustellen.

9. Robotisches Anbaugerät nach Anspruch 7 oder 8, wobei das robotische Anbaugerät (6) dazu eingerichtet ist, in Antwort auf ein vom Empfänger (242) empfangenes Signal, ein Navigationssignal an der mechatronischen Schnittstelle (206) bereitzustellen.

10. Robotisches Anbaugerät (6) nach einem der Ansprüche 7 bis 9, aufweisend wenigstens einen Sender (244, 246) zum Senden elektromagnetischer Strahlung zum Senden von Signalen an ein OP-Navigationssystem (60), wobei der Sender vorzugsweise eine IR-Lichtquelle aufweist.

11. System mit einer Haltevorrichtung (1) nach wenigstens einem der vorstehenden Ansprüche 1 bis 6 und einem am distalen Ende (4) aufgenommenen Anbaugerät (6), wobei das Anbaugerät (6) wenigstens einen Sender zum Senden elektromagnetischer Strahlung zum Senden von Signalen an ein OP-Navigationssystem (60) aufweist, und/oder wenigstens eine Navigationskamera (250) zum Erfassen eines Operationsfelds aufweist.

12. Verfahren zur Kommunikation zwischen einer Haltevorrichtung (1) nach einem der Ansprüche 1 bis 6 und einem OP-Navigationssystem (60), mit den Schritten:
- Empfangen von elektromagnetischer Strahlung von einem OP-Navigationssystem (60) mittels des Empfängers (70);
- Senden eines entsprechenden Signals von dem Empfänger (70) an die Steuereinheit (64);
- Verarbeiten, mittels der Steuereinheit (64), des von dem Empfänger (70) empfangenen Signals; und
- Versetzen der Haltevorrichtung (1) in einen Navigationsmodus.

13. Verfahren nach Anspruch 12, wobei der Empfänger (70) der Haltevorrichtung (1) wenigstens ein 3D-Magnetometer aufweist, und die Haltevorrichtung in einem elektromagnetischen Feld eines OP-Navigationssystems (60) angeordnet ist, umfassend die Schritte:
- Bestimmen, basierend auf von dem wenigstens einen 3D-Magnetometer gesendeten Signalen, einer relativen Pose der Haltevorrichtung (1) bezogen auf das elektromagnetische Feld;
- Bestimmen einer absoluten Pose der Haltevorrichtung (1);
- Vergleichen der relativen und der absoluten Pose der Haltevorrichtung (1); und
- Bestimmen eines Fehlers, mittels einer Fehlerkorrektureinheit, des elektromagnetischen Felds basierend auf dem Vergleich der relativen und der absoluten Pose der Haltevorrichtung (1) und vorzugsweise ferner umfassend den Schritt:
- Bestimmen einer Position von einem im elektromagnetischen Feld befindlichen Objekt unter Verwendung des bestimmten Fehlers.

14. Verfahren nach einem der Ansprüche 12 oder 13, aufweisend die Schritte:
- Erfassen mittels einer an der Haltevorrichtung (1) angeordneten Navigationskamera (250) eines durch die Haltevorrichtung (1) für das OP-Navigationssystem abgeschatteten Bereichs;
- Bereitstellen von durch die Navigationskamera (250) erfassten optischen Daten an dem OP-Navigationssystem (60, 66); und
- Zusammenfügen von durch das OP-Navigationssystem (60, 66) erfassten optischen Daten und den durch die Navigationskamera (250) erfassten optischen Daten zum Erhalten eines Bildes ohne einen durch die Haltevorrichtung (1) abgeschatteten Bereich.

15. Computerprogramm mit Programmcode-Mitteln, die einen Prozessor einer Haltevorrichtung (1) nach einem der vorhergehenden Ansprüche 1 bis 6 zur Ausführung der Schritte des Verfahrens nach einem der Ansprüche 12 bis 14 veranlassen, wenn diese Programmcode-Mittel von dem Prozessor ausgeführt werden.

## Claims

1. A holding device (1), particularly a holding arm, for medical purposes for holding an attached device, particularly a surgical mechatronic assistance system and/or surgical instrument, having
a proximal end (2) for attaching the holding device (1) to a base and a distal end (4) for receiving an attached device (6);
at least one first and one second arm segment (12, 14), wherein the first arm segment (12) being connected to a first joint (13) and the second arm segment (14) being connected to a second joint (15), wherein each joint (13, 15) being releasable and lockable;
an operator control device (50) for releasing and/or locking the corresponding joint (13, 15) for placing the holding device (1) in a desired pose; and
a control unit (64) comprising a processor and program code means for controlling the holding device (1);
at least one receiver (70) for electromagnetic radiation connected to the control unit (64) and adapted for transmitting signals to the control unit (64) on the basis of received electromagnetic signals of a surgical navigation system (60),
**characterized in that** the holding device (1) is adapted to be operated in a navigation mode and an operator mode,
wherein the holding device (1) in the operator mode can be operated purely manually by means of an operator control device (50) provided for the holding device (1), and in the navigation mode is prepared and adapted for receiving commands from the navigation system and being controlled by it,
wherein the control unit (64) is set up for determining on the basis of the signals being transmitted from the receiver to the control unit, whether the holding device (1) is present in a navigated surgical environment or is not present in a navigated surgical environment,
and wherein the holding device (1) switches automatically into the operator mode, if it is not present in a navigated surgical environment, and switches automatically into the navigation mode, if it is present in a navigated surgical environment.

2. The holding device (1) according to claim 1, further comprising at least one transmitter for transmitting electromagnetic radiation for transmitting signals to a surgical navigation system (60), wherein the transmitter preferably comprises an IR light source (124).

3. The holding device (1) according to claim 2, comprising a first display unit (34) disposed on the first joint (13) and a second display unit (36) disposed on the second joint (15), wherein the first and/or second display unit (34, 36) each comprise at least one IR light source (124) and are set up for displaying at least one status of the holding device (1) and/or a status of an attached device (6).

4. The holding device (1) according to claim 2 or 3, wherein the one or more transmitters is/are set up for for emitting a signal, if one or more brakes on the joints (11, 13, 15, 17, 19, 21, 23) are opened and/or one or more joints (11, 13, 15, 17, 19, 21, 23) of the holding device (1) are moved.

5. The holding device (1) according to any of the preceding claims, further comprising a navigation camera for capturing an operating field, wherein the holding device is set up for providing signals captured by the navigation camera to an interface for the surgical navigation system in processed or unprocessed form.

6. The holding device (1) according to any one of the preceding claims, further comprising a BUS system and a first mechatronic interface at the proximal end and a second mechatronic interface at the distal end, wherein the mechatronic interface at the distal end is provided for coupling to a robotic attached device.

7. A robotic attached device (6), particularly a manipulator device, having:
a frame;
a drive supported on the frame,
an instrument receptacle (220) for holding a surgical instrument (202), which can be driven by means of the drive, and
a mechatronic interface (206) for coupling to a distal interface (8) of a holding device (1) according to any one of the preceding claims,
**characterized by** at least one receiver (242) for electromagnetic radiation connected to a robot control unit and adapted for transmitting signals to the robot control unit on the basis of received electromagnetic signals of an surgical navigation system (60),
wherein the robotic attached device (6) can be operated in a robot navigation mode and a robot operator mode, wherein the robotic attached device (6) in the robot operator mode can be operated purely manually by means of an robot operator control unit provided for the robotic attached device (6), and in the robot navigation mode is prepared for receiving commands from the navigation system (60),
wherein the robot control unit is set up for determining on the basis of the signals being transmitted from the receiver (242) to the robot control unit, whether the attached device (6) is present in a navigated surgical environment or is not present in a navigated surgical environment,
and wherein the attached device (6) switches automatically into the robot operator mode, if it is not present in a navigated surgical environment, and switches automatically into the robot navigation mode, if it is present in a navigated surgical environment.

8. The robotic attached device according to claim 7, **characterized by** a navigation camera (250) for capturing an operating field, wherein the robotic attached device (6) is set up for providing signals captured by the navigation camera (250) to an interface for the surgical navigation system and/or the mechatronic interface (206) in a processed or unprocessed form.

9. The robotic attached device according to claim 7 or 8, wherein the robotic attached device (6) is set up for providing a navigation signal to the mechatronic interface (206) in response to a signal received by the receiver (242).

10. The robotic attached device (6) according to any one of the claims 7 through 9, comprising at least one transmitter (244, 246) for transmitting electromagnetic radiation for transmitting signals to a surgical navigation system (60), wherein the transmitter preferably comprises an IR light source.

11. A system having a holding device (1) according to at least one of the preceding claims 1 through 6 and an attached device (6) received at a distal end (4), wherein the attached device (6) comprises at least one transmitter for transmitting electromagnetic radiation for transmitting signals to a surgical navigation system (60), and/or at least one navigation camera (250) for capturing an operating field.

12. A method for communicating between a holding device (1) according to any one of the claims 1 through 6 and a surgical navigation system (60), having the steps:
- receiving electromagnetic radiation from a surgical navigation system (60) by means of the receiver (70);
- transmitting a corresponding signal from the receiver (70) to the control unit (64);
- processing the signal received by the receiver (70) by means of the control unit (64); and
- switching the holding device (1) into a navigation mode.

13. The method according to claim 12, wherein the receiver (70) of the holding device (1) comprises at least one 3D magnetometer and the holding device is disposed in an electromagnetic field of a surgical navigation system (60), comprising the steps:
- determining a relative pose of the holding device (1) relative to the electromagnetic field on the basis of signals transmitted by the at least one 3D magnetometer;
- determining an absolute pose of the holding device (1);
- comparing the relative and absolute pose of the holding device (1); and
- determining an error of the electromagnetic field by means of an error correcting unit, based on the comparison of the relative and the absolute pose of the holding device (1), and preferably further comprising the step:
- determining a position of an object present in the electromagnetic field using the determined error.

14. The method according to claim 12 or 13, comprising the steps:
- capturing a region obscured for the surgical navigation system by the holding device (1) by means of a navigation camera (250) disposed on the holding device (1);
- providing optical data captured by the navigation camera (250) to the surgical navigation system (60, 66); and
- merging optical data captured by the surgical navigation system (60, 66) and the optical data captured by the navigation camera (250) for retaining an image without a region obscured by the holding device (1).

15. A computer program having program code means causing a processor of a holding device (1) according to any one of the preceding claims 1 through 6 to execute the steps of the method according to any one of the claims 12 through 14 when said program code means are executed by the processor.

## Revendications

1. Dispositif de retenue (1), en particulier bras de retenue, pour des fins médicales pour tenir un accessoire, en particulier un système d'assistance mécatronique chirurgical et/ou un instrument chirurgical, avec
une extrémité proximale (2) pour fixer le dispositif de retenue (1) à une base et une extrémité distale (4) pour accueillir un accessoire (6);
au moins un premier et un deuxième segments de bras (12, 14), le premier segment de bras (12) étant relié à une première articulation (13) et le deuxième segment de bras (14) étant relié à une deuxième articulation (15), chaque articulation (13, 15) pouvant être libérée et bloquée;
un dispositif de commande (50) pour libérer et/ou bloquer l'articulation (13, 15) correspondante afin d'amener le dispositif de retenue (1) dans une pose souhaitée; et
une unité de commande (64), comprenant un processeur et des moyens de code de programme, pour commander le dispositif de retenue (1);
au moins un récepteur (70) de rayonnement électromagnétique qui est connecté à l'unité de commande (64) et adapté pour envoyer des signaux à l'unité de commande (64) sur la base de signaux électromagnétiques reçus d'un système de navigation chirurgical (60),
**caractérisé en ce que** le dispositif de retenue (1) est adapté pour pouvoir être utilisé dans un mode de navigation et dans un mode de commande,
dans lequel le dispositif de retenue (1), dans le mode de commande, peut être commandé de manière purement manuelle par l'intermédiaire d'une unité de commande (50) prévue pour le dispositif de retenue (1), et est préparé et configuré pour, dans le mode de navigation, recevoir des instructions du système de navigation et être commandé par celui-ci,
dans lequel l'unité de commande (64) est adaptée pour déterminer, sur la base des signaux envoyés par le récepteur à l'unité de commande, si le dispositif de retenue (1) se trouve ou non dans un environnement chirurgical navigué,
et dans lequel le dispositif de retenue (1) passe automatiquement en mode de commande s'il ne se trouve pas dans un environnement opératoire navigué et passe automatiquement en mode de navigation s'il se trouve dans un environnement chirurgical navigué.

2. Dispositif de retenue (1) selon la revendication 1, comprenant en outre au moins un émetteur pour émettre un rayonnement électromagnétique pour envoyer des signaux à un système de navigation chirurgical (60), l'émetteur comprenant de préférence une source de lumière infrarouge (124).

3. Dispositif de retenue (1) selon la revendication 2, comprenant une première unité d'affichage (34) qui est disposée sur la première articulation (13) et une deuxième unité d'affichage (36) qui est disposée sur la deuxième articulation (15), dans lequel la première et/ou la deuxième unité(s) d'affichage (34, 36) comporte(nt) chacune au moins une source de lumière infrarouge (124) et est/sont adaptée(s) pour indiquer au moins un état du dispositif de retenue (1) et/ou un état d'un accessoire (6).

4. Dispositif de retenue (1) selon la revendication 2 ou 3, dans lequel le ou les plusieurs émetteurs est/sont conçu(s) pour émettre un signal lorsqu'un ou plusieurs freins sur les articulations (11, 13, 15, 17, 19, 21, 23) sont ouverts et/ou lorsqu'une ou plusieurs articulations (11, 13, 15, 17, 19, 21, 23) du dispositif de retenue (1) sont déplacées.

5. Dispositif de retenue (1) selon l'une quelconque des revendications précédentes, présentant en outre une caméra de navigation pour capturer un champ opératoire, dans le lequel le dispositif de retenue est configuré pour mettre à disposition, sur une interface pour le système de navigation chirurgical, des signaux acquis par la caméra de navigation, traités ou non traités.

6. Dispositif de retenue (1) selon l'une quelconque des revendications précédentes, comprenant en outre un système BUS ainsi qu'une première interface mécatronique à l'extrémité proximale et une deuxième interface mécatronique à l'extrémité distale, dans lequel l'interface mécatronique à l'extrémité distale est prévue pour le couplage avec un accessoire robotique.

7. Accessoire robotique (6), en particulier dispositif de manipulation, avec:
un cadre;
un entraînement soutenu par le cadre,
un porte-instruments (220) pour tenir un instrument chirurgical (202), qui peut être entraîné au moyen de l'entraînement, et
une interface mécatronique (206) destinée à être couplée à une interface distale (8) d'un dispositif de retenue (1) selon l'une quelconque des revendications précédentes,
**caractérisé par** au moins un récepteur (242) de rayonnement électromagnétique qui est connecté à une unité de commande de robot et adapté pour envoyer des signaux à l'unité de commande de robot sur la base de signaux électromagnétiques reçus d'un système de navigation chirurgical (60),
dans lequel l'accessoire robotique (6) peut fonctionner dans un mode de navigation robotique et dans un mode de commande robotique, dans lequel, dans le mode de commande robotique, l'accessoire robotique (6) peut être commandé de manière purement manuelle par l'intermédiaire d'une unité de commande robotique prévue pour l'accessoire (6), et, dans le mode de navigation robotique, il est préparé pour recevoir des instructions du système de navigation (60),
dans lequel l'unité de commande de robot est adaptée pour déterminer, sur la base des signaux envoyés par le récepteur (242) à l'unité de commande de robot, si l'accessoire (6) se trouve dans un environnement chirurgical navigué ou non, et dans lequel l'accessoire (6) passe automatiquement en mode de commande de robot s'il ne se trouve pas dans un environnement chirurgical navigué et passe automatiquement en mode de navigation de robot s'il se trouve dans un environnement chirurgical navigué.

8. Accessoire robotique selon la revendication 7, **caractérisé par** une caméra de navigation (250) pour capturer un champ opératoire, l'accessoire robotique (6) étant configuré pour mettre à disposition des signaux acquis par la caméra de navigation (250), traités ou non traités, sur une interface pour le système de navigation chirurgical et/ou l'interface mécatronique (206).

9. Accessoire robotique selon la revendication 7 ou 8, dans lequel l'accessoire robotique (6) est configuré pour fournir un signal de navigation à l'interface mécatronique (206) en réponse à un signal reçu du récepteur (242).

10. Accessoire robotique (6) selon l'une quelconque des revendications 7 à 9, comprenant au moins un émetteur (244, 246) pour émettre un rayonnement électromagnétique pour envoyer des signaux à un système de navigation chirurgical (60), l'émetteur comprenant de préférence une source de lumière infrarouge.

11. Système comprenant un dispositif de retenue (1) selon au moins l'une des revendications 1 à 6 précédentes et un accessoire (6) accueilli à l'extrémité distale (4), dans lequel l'accessoire (6) présente au moins un émetteur pour émettre un rayonnement électromagnétique pour envoyer des signaux à un système de navigation chirurgical (60), et/ou présentant au moins une caméra de navigation (250) pour capturer un champ opératoire.

12. Procédé de communication entre un dispositif de retenue (1) selon l'une quelconque des revendications 1 à 6 et un système de navigation chirurgical (60), comprenant les étapes suivantes:
- recevoir un rayonnement électromagnétique provenant d'un système de navigation chirurgicale (60) au moyen du récepteur (70);
- envoyer un signal correspondant du récepteur (70) à l'unité de commande (64);
- traiter, au moyen de l'unité de commande (64), le signal reçu du récepteur (70); et
- mettre le dispositif de retenue (1) en un mode de navigation.

13. Procédé selon la revendication 12, dans lequel le récepteur (70) du dispositif de retenue (1) comporte au moins un magnétomètre 3D, et le dispositif de retenue est disposé dans un champ électromagnétique d'un système de navigation chirurgical (60), comprenant les étapes suivantes:
- déterminer, sur la base de signaux envoyés par le, au moins un, magnétomètre 3D, une pose relative du dispositif de retenue (1) par rapport au champ électromagnétique;
- déterminer une pose absolue du dispositif de retenue (1);
- comparer la pose relative avec la pose absolue du dispositif de retenue (1); et
- déterminer une erreur au moyen d'une unité de correction d'erreur, du champ électromagnétique sur la base de la comparaison de la pose relative avec la pose absolu du dispositif de retenue (1), et comprenant en outre, de préférence, l'étape consistant à:
- déterminer une position d'un objet situé dans le champ électromagnétique par utilisation de l'erreur déterminée.

14. Procédé selon l'une quelconque des revendications 12 ou 13, comprenant les étapes consistant à:
- capturer, au moyen d'une caméra de navigation (250) disposée sur le dispositif de retenue (1), une zone obstrue par le dispositif de retenue (1) pour le système de navigation chirurgical;
- fournir des données optiques acquises par la caméra de navigation (250) au système de navigation chirurgicale (60, 66); et
- fusionner les données optiques acquises par le système de navigation chirurgical (60, 66) et les données optiques acquises par la caméra de navigation (250) pour obtenir une image sans zone obstrue par le dispositif de retenue (1).

15. Programme informatique comprenant des moyens de code de programme, qui amènent un processeur d'un dispositif de retenue (1) selon l'une quelconque des revendications 1 à 6 précédentes à exécuter les étapes du procédé selon l'une quelconque des revendications 12 à 14, lorsque ces moyens de code de programme sont exécutés par ledit processeur.
